# EUROPÄISCHE PATENTSCHRIFT

(11) **EP 3 096 723 B1**
(45) Veröffentlichungstag und Bekanntmachung des Hinweises auf die Patenterteilung: **11.10.2023**
(21) Anmeldenummer: 15703482.8
(22) Anmeldetag: 23.01.2015
(51) Int. Cl.: A61F 7/08, A61F 7/02

(54) **WÄRMEEINRICHTUNG MIT EINEM LATENTWÄRMESPEICHERMITTEL**
HEAT DEVICE HAVING A LATENT-HEAT STORAGE MEANS
DISPOSITIF CHAUFFANT COMPORTANT UN MOYEN D'ACCUMULATION DE CHALEUR LATENTE

(30) Priorität: 23.01.2014 DE 102014000941; 26.01.2014 DE 102014000799; 09.04.2014 DE 102014005167; 21.05.2014 DE 102014007514
(43) Veröffentlichungstag der Anmeldung: 30.11.2016
(73) Patentinhaber: WFI Wärmflascheninnovation UG (Haftungsbeschränkt), 81241 München (DE)
(72) Erfinder: OHMER, Benjamin, 80333 München (DE)
(74) Vertreter: Kehl, Ascherl, Liebhoff & Ettmayr Patentanwälte Partnerschaft mbB
(86) Internationale Anmeldenummer: PCT/EP2015/051342
(87) Internationale Veröffentlichungsnummer: WO 2015/110574

(56) Entgegenhaltungen:
- WO-A1-2007/117159
- WO-A1-2009/139877
- US-A1- 2004 186 541
- US-A1- 2008 255 644
- US-A1- 2012 305 231
- Anonymous: "Sodium acetate - Wikipedia", , 29 December 2013 (2013-12-29), XP055721869, Retrieved from the Internet: URL:https://en.wikipedia.org/w/index.php?t itle=Sodium_acetate&oldid=588269360 [retrieved on 2020-08-12]

## Beschreibung

Die vorliegende Erfindung bezieht sich auf den Gegenstand der anhängigen unabhängigen Ansprüche 1, 5 und 6. Weitere Ausführungsformen sind in den abhängigen Ansprüchen offenbart.

Wärmeeinrichtungen, wie Wärmflaschen und Körnerkissen, gibt es seit vielen Jahren. Derartige Wärmeeinrichtungen weisen jedoch stets das Problem auf, dass sie von den Nutzern zu heiß befüllt werden können oder mittels einer Heizeinrichtung, wie einem Ofen, zu sehr erhitzt werden. Die hohe Hitze führt dazu, dass sich Verbrennungen der Haut ergeben können. Um Verbrennungen zu vermeiden wurde der "Hot Water Bottles Safety Standard BS 1970" eigeführt, der durch den "Hot Water Bottles Safety Standard BS 1970:2012" im Jahr 2012 erneuert wurde. Gemäß diesem Standard müssen Wärmflaschen mit einer bestimmten Wanddicke hergestellt werden, damit der Wärmeübertrag begrenzt wird. Ferner müssen gemäß diesem Standard Wärmflaschen mit dem Hinweis versehen werden, dass kein kochendes Wasser in sie eingefüllt werden darf.

Weiterhin gibt es Schutzrechtsschriften, in denen sich die Erfinder dem Problem der auftretenden Verbrennungen angenommen haben. So offenbart z.B. die deutsche Gebrauchsmusterschrift DE 77 07 739 die unterschiedliche Gestaltung der Wandungen der Wärmflasche, wodurch unterschiedlich stark Wärme übertragen wird. Ferner offenbart die Druckschrift DE 85 04 306 U1 eine Wärmflasche, bei der auf der einen Seite eine Isolationsschicht angeordnet ist. Ferner offenbart die Druckschrift DE 69101 711T2 eine Wärmflasche, bei der die Seiten der Wärmflasche unterschiedlich beflockt sind.

Weiterer relevanter Stand der Technik ist in US 2004/186541 A1, US 2008/255644 A1, WO 2009/139877 A1, WO 2007/117159 A1, US 2012/305231 A1 und auf Wikipedia unter dem Titel "Sodium acetate" offenbart.

Alle hier bisher genannten Ansätze sind mangelhaft, da die Mehrheit der Nutzer zum einen stets das Wasser für die Wärmflasche mittels eines Wasserkochers erhitzen. Da Wasserkocher in der Regel erst dann abschalten, wenn das Wasser kocht verwenden die meisten Personen das frisch gekochte Wasser zum Einfüllen in die Wärmflasche, wodurch es gefährlich heiß sein kann.

Zum anderen versuchen viele Nutzer mit dem Einfüllen von sehr heißem Wasser zu erreichen, dass das Wasser möglichst lange warm bleibt, um möglichst lange die Wärmflasche nutzen zu können. Hierdurch wird ein weiteres Problem der bekannten Wärmflaschen erkennbar. Bekannte Wärmflaschen weisen eine im Wesentlichen kontinuierliche Abkühlrate auf, d.h., dass die Wärmflasche über die Gesamtnutzungszeit gesehen größtenteils zu heiß oder zu kalt ist.

Es ist somit die Aufgabe der vorliegenden Erfindung eine Wärmeeinrichtung bereitzustellen, welche die zuvor genannten Probleme abmildert bzw. eine Komforterhöhung bei der Verwendung einer solchen Wärmeeinrichtung schafft.

Die zuvor genannte Aufgabe wird erfindungsgemäß durch eine Wärmeeinrichtung, insbesondere eine Wärmflasche oder ein Wärmekissen, zum zumindest mittelbaren in Kontakt bringen mit einem Lebewesen, insbesondere einem Menschen und/oder einem Tier, gelöst. Die Wärmeeinrichtung umfasst hierbei die Merkmale des Anspruchs 1.

Diese Lösung ist vorteilhaft, da zum einen überschüssige Wärme dem fließfähigen Material zunächst für eine spätere Nutzung entzogen wird, wodurch die Gefahr an Verbrennungen reduziert wird und zum anderen durch das spätere Freigeben der gespeicherten Wärme eine Komfortverbesserung erreicht wird. Das Phasenwechselmaterial bewirkt durch den teilweisen oder vollständigen endothermen Zustandswechsel somit eine Abkühlung des fließfähigen Materials, wobei die Wärme in einem späteren Zeitraum bevorzugt konstant oder bevorzugt im Wesentlichen konstant an das fließfähige Material und/oder an die Wandung abgegeben wird. Es wurde experimentell nachgewiesen, dass durch die erfindungsgemäße Vorrichtung nicht nur eine deutlich homogenere Wärmeabgabe erreicht wird, sondern ebenfalls die Dauer der Wärmeabgabe, insbesondere in einem optimalen Wärmebereich zwischen 38°C und 48°C, verlängert wird. Die erfindungsgemäße Wärmeeinrichtung ist somit im Vergleich mit einer baugleichen Wärmeeinrichtung, insbesondere Wärmflasche, die kein Latentwärmespeichermittel aufweist, bei gleichen Randbedingungen, insbesondere Temperatur des fließfähigen Mediums, Menge des fließfähigen Mediums und Umgebungstemperatur länger warm, insbesondere länger in einem optimalen Temperaturbereich. Dies erklärt sich durch eine starke Reduzierung der in den ersten Minuten nach außen abgegebenen Wärmemenge, die durch eine Aufnahme von Wärme durch das Latentwärmespeichermittel bewirkt wird. Je höher die Temperaturdifferenz zwischen der Umgebung und der Wärmeeinrichtung ist, desto größer ist die Wärmeabgabe an die Umgebung. Da durch die erfindungsgemäße Wärmeeinrichtung zunächst eine deutliche Abkühlung der fließfähigen Substanz durch das Speichern eines Teils der Wärmemenge der fließfähigen Substanz in dem Latentwärmespeichermittel erfolgt, sinkt auch die je Sekunde an die Umgebung abgegebene Wärmemenge, wodurch die Wärme dann über einen längeren Zeitraum abgegeben werden kann.

Die Temperierung des fließfähigen Materials kann eine Abkühlung des fließfähigen Materials bedeuten oder bei der Abkühlung des fließfähigen Materials erfolgen, wobei die Temperierung des fließfähigen Materials bzw. fließfähigen Stoffs, was auch ein Materialgemisch oder Stoffgemisch sein kann, bevorzugt zumindest das Zuführen von Wärme zu dem fließfähigen Material bedeutet, wobei die Wärme bevorzugt das Erhitzen des fließfähigen Materials, das Halten der Temperatur des fließfähigen Materials oder die Reduzierung einer Abkühlgeschwindigkeit des fließfähigen Materials bewirkt.

In anderen Worten: das Latentwärmespeichermittel weist bevorzugt ein Phasenwechselmaterial, insbesondere Natriumacetat, auf, wobei das Phasenwechselmaterial bei einer ersten Zustandsänderung infolge einer Erwärmung Energie aufnimmt und bei einer zweiten Zustandsänderung Energie in Form von Wärme abgibt. Phasenwechselmaterialien weisen Natriumacetat-Trihydrat bzw. Natriumacetat oder zusätzlich Paraffine oder Mischungen aus diesen Materialien auf. Natriumacetat-Trihydrat weist bevorzugt eine Schmelztemperatur von im Wesentlichen oder genau 58°C auf, Paraffin, insbesondere Hartparaffin, weist bevorzugt eine Schmelztemperatur von ca. oder genau 60°C auf. Das Phasenwechselmaterial weist bevorzugt eine Schmelztemperatur von mehr als 36°C, insbesondere von mehr als 40°C, insbesondere von mehr als 42°C, insbesondere von mehr als 45°C,insbesondere von mehr als 47°C,insbesondere von mehr als 50°C,insbesondere von mehr als 52°C, insbesondere von mehr als 55°C, insbesondere von mehr als 56°C, wie z.B. bis zu 60°C oder bis zu 65°C oder bis zu 70°C oder bis zu 80°C auf. Somit wird gemäß der vorliegenden Erfindung bevorzugt ein Phasenwechselmaterial verwendet, dessen Schmelztemperatur zwischen 30°C und 80°C und bevorzugt zwischen 36°C und 70°C und besonders bevorzugt zwischen 40°C und 65°C, insbesondere zwischen 45°C und 63°C, liegt. Das Phasenwechselmaterial weist bevorzugt zumindest Salzbestandteile auf und besteht besonders bevorzugt zu überwiegenden Massenanteilen aus einem oder mehreren Salzen.

Gemäß einer nicht beanspruchten Ausführungsform ist das Latentwärmespeichermittel derart ausgebildet, dass bei einer definierten Menge an definiert temperiertem fließfähigem Material das Phasenwechselmaterial die endotherme Zustandsänderung vollständig erfährt, wobei bevorzugt ebenfalls die Abkühlrate des fließfähigen Materials definiert ist. Diese Ausführungsform ist vorteilhaft, da durch den vollständigen Zustandswechsel ein stabiler Zustand erreicht wird, in dem die aufgenommene Wärme gespeichert und jederzeit abrufbar ist.

Gemäß einer nicht beanspruchten Ausführungsform weist das Latentwärmespeichermittel ein Betätigungsmittel, insbesondere einen bevorzugt mindestens ein Metall aufweisenden Knackfrosch, zum Auslösen der exothermen Zustandsänderung auf.

Ist z.B. die Temperatur des fließfähigen Materials unter die beim Phasenübergang bzw. bei der exothermen Zustandsänderung sich ergebenden Temperatur gefallen, z.B. auf Raumtemperatur, so kann mittels des Betätigungsmittels die exotherme Zustandsänderung des Phasenwechselmaterials ausgelöst werden, wodurch eine Erwärmung des fließfähigen Materials bewirkt wird.

Das Betätigungsmittel ist bevorzugt teilweise oder vollständig von Phasenwechselmaterial umgeben bzw. steht mit diesem bevorzugt unmittelbar in Kontakt.

Gemäß einer weiteren bevorzugten Ausführungsform der vorliegenden Erfindung ist das Latentwärmespeichermittel bevorzugt derart ausgebildet, dass bei einer definierten Menge an definiert temperiertem fließfähigem Material das Phasenwechselmaterial die endotherme Zustandsänderung teilweise erfährt, wobei bevorzugt ebenfalls die Abkühlrate des fließfähigen Materials definiert ist.

Bei einer Menge A an fließfähigem Material, insbesondere Wasser, einer Temperatur T des fließfähigen Materials und bevorzugt einer Abkühlrate R des fließfähigen Materials weist das Latentwärmespeichermittel somit bevorzugt eine dreidimensionale äußere Form und bevorzugt eine Menge darin angeordneten Phasenwechselmaterials auf, wobei die Menge an Phasenwechselmaterial in Abhängigkeit von der dreidimensionalen äußeren Form des Latentwärmespeichermittels derart gewählt ist, dass das Phasenwechselmaterial die endotherme Zustandsänderung nur teilweise erfährt. Bevorzugt kann hierbei z.B. die Regel angewendet werden, dass bei dergleichen Menge A an fließfähigem Material, dergleichen Temperatur T und bevorzugt der gleichen Abkühlrate R mit zunehmender Oberfläche der dreidimensionalen äußeren Form des Latentwärmespeichermittels die Menge an Phasenwechselmaterial ebenfalls zunehmen muss.

Beim Abkühlen des fließfähigen Materials z.B. unter die Temperatur, bei der das Phasenwechselmaterial die Phase bzw. den Zustand wechselt, erfolgt durch das Phasenwechselmaterial eine Wärmeabgabe und dadurch eine Rückgängigmachung der teilweise erfolgten endothermen Zustandsänderung. Das Phasenwechselmaterial gibt bei der Rückgängigmachung die zuvor aufgenommen Energie bevorzugt im Wesentlichen bzw. vollständig in Form von Wärme an das fließfähige Material ab.

Phasenwechselmaterial, das nicht vollständig die Phase gewechselt hat, neigt bevorzugt dazu den teilweise erfolgten Phasenwechsel rückgängig zu machen, wodurch das Phasenwechselmaterial Wärme abgibt. Phasenwechselmaterial, das hingegen vollständig die Phase gewechselt hat, wechselt die Phase bzw. den erreichten Zustand erst nach einem Auslöseereignis, das z.B. mittels des Betätigungselements erzeugt bzw. gestartet werden kann.

Diese Ausführungsform stellt somit in vorteilhafter Weise eine Art Wärmepuffer dar.

Im Falle einer Wärmeeinrichtung, der das fließfähige Material temperiert zugeführt wird, wie z.B. im Falle einer Wärmflasche, nimmt das Phasenwechselmaterial zunächst Wärme von dem fließfähigen Material auf, wodurch das fließfähige Material heruntergekühlt wird. Da die Temperatur des erhitzten fließfähigen Materials von den Nutzern der Wärmeeinrichtung oft nicht auf eine Idealtemperatur (z.B. 50°-60°C) eingestellt wird, sondern oftmals deutlich heißer ist (z.B. 95°C) ergeben sich leicht Verletzungen bzw. Verbrennungen der Haut. Durch das Herunterkühlen der Temperatur des fließfähigen Materials mittels dem Phasenwechselmaterial erfolgt eine Speicherung der überschüssigen Wärme. Die gespeicherte Wärme wird anschließend wieder von dem Phasenwechselmaterial abgegeben sobald die Temperatur des fließfähigen Materials unter eine Schwellentemperatur fällt. Die von dem Phasenwechselmaterial abgegebene Wärme bewirkt dann bevorzugt, dass die Temperatur des fließfähigen Materials im Wesentlichen im Bereich der Schwellentemperatur konstant bzw. im Wesentlichen konstant gehalten wird oder die von dem Phasenwechselmaterial abgegebene Wärme bewirkt zumindest, dass das Abkühlen des fließfähigen Materials langsamer erfolgt.

Gemäß einer weiteren bevorzugten Ausführungsform der vorliegenden Erfindung ist an dem Aufnahmeraum eine verschließbare Zuführ- und Entleeröffnung zum Zuführen des, insbesondere erhitzten, fließfähigen Materials zu dem Aufnahmeraum und zum Ausleiten des, insbesondere erkalteten, fließfähigen Materials aus dem Aufnahmeraum heraus ausgebildet.

Diese Ausführungsform ist vorteilhaft, da das fließfähige Material außerhalb der Wärmeeinrichtung erhitzbar bzw. temperierbar ist.

Gemäß einer weiteren bevorzugten Ausführungsform der vorliegenden Erfindung ist der Aufnahmeraum derart gekapselt ausgebildet, dass das fließfähige Material dauerhaft in dem Aufnahmeraum verbleibt. Dies bedeutet besonders bevorzugt, dass das fließfähige Material nur durch eine Beschädigung oder Zerstörung des Aufnahmeraums aus dem Aufnahmeraum entnehmbar ist. Gemäß dieser Ausführungsform ist insbesondere keine Zuführ- und/oder Entleeröffnung vorgesehen. Diese Ausführungsform ist vorteilhaft, da das Latentwärmespeichermittel und das fließfähige Material zeitgleich durch dieselbe Wärmequelle, z.B. ein Backofen, ein Mikrowellenherd, ein Kochtopf, etc. erhitzbar ist. Es müssen somit keine aufwendigen Austauschtätigkeiten durchgeführt werden und durch das Nichtvorhandensein einer Verschlusseinrichtung kann eine solche nicht auf ungenügende Weise verschlossen werden, wodurch eine hohe Sicherheit bei der Benutzung gegeben ist.

Gemäß einer weiteren bevorzugten Ausführungsform der vorliegenden Erfindung ist das mindestens oder genau eine Latentwärmespeichermittel innerhalb des Aufnahmeraums oder an der Wandung des Aufnahmeraums angeordnet oder ist als Teil der Wandung ausgebildet.

Bevorzugt ist das Latentwärmespeichermittel im Bereich der Wandung oder an der Wandung, insbesondere innerhalb des Aufnahmeraum oder außerhalb des Aufnahmeraums, angeordnet.

Gemäß einer weiteren bevorzugten Ausführungsform der vorliegenden Erfindung liegt das Verhältnis zwischen der Menge oder Masse des in dem Aufnahmeraum aufnehmbaren fließfähigen Materials und der Menge oder Masse des Phasenwechselmaterials zwischen 2:1 und 7:1, bevorzugt zwischen 2,5:1 und 5,5:1 und besonders bevorzugt zwischen 2,5:1 und 4,5:1. Bevorzugt liegt somit das Verhältnis zwischen der Menge des in dem Aufnahmeraum aufnehmbaren fließfähigen Materials und der Menge des Phasenwechselmaterials zwischen 2:1 und 6:1, bevorzugt zwischen 2,5:1 und 5,5:1 und besonders bevorzugt zwischen 2,5:1 und 4,5:1 liegt oder das Verhältnis zwischen der Masse des in dem Aufnahmeraum aufnehmbaren fließfähigen Materials und der Masse des Phasenwechselmaterials liegt zwischen 2:1 und 6:1, bevorzugt zwischen 2,5:1 und 5,5:1 und besonders bevorzugt zwischen 2,5:1 und 4,5:1. Im Sinne der vorliegenden Erfindung kann das Verhältnis zwischen der Menge oder Masse des in dem Aufnahmeraum aufnehmbaren fließfähigen Materials und der Menge oder Masse des Phasenwechselmaterials auch zwischen 2,5:1 und 4:1, bevorzugt zwischen 2,5:1 und 3,5:1 und besonders bevorzugt zwischen 2:1 und 4:1 liegen.

Gemäß einer weiteren bevorzugten Ausführungsform der vorliegenden Erfindung sind die Wandungen der Wärmeeinrichtung unterschiedlich isolierend ausgebildet. Bevorzugt ist eine mit dem Körper eines Lebewesens in Kontakt bringbare Wandung weniger stark isoliert als eine durch den Aufnahmeraum davon beabstandete Wandung. Diese Ausführungsform ist vorteilhaft, da die Wärme, die an die Umgebung abgegeben wird, reduziert wird und so eine Verlängerung der Dauer bewirkt wird, in der an das Lebewesen Wärme abgegeben wird und in der das Latentwärmespeichermittel Wärme von dem fließfähigen Material aufnehmen kann.

Die vorliegende Erfindung bezieht sich ebenfalls auf eine Wärmeeinrichtung, insbesondere auf eine Wärmflasche oder ein Wärmekissen, zum zumindest mittelbaren in Kontakt bringen mit einem Lebewesen, die bevorzugt mindestens einen Aufnahmeraum zum Vorhalten eines fließfähigen Materials umfasst, wobei der Aufnahmeraum zumindest abschnittsweise durch eine flexible Wandung ausgebildet ist, wobei die flexible Wandung zumindest mittelbar mit dem Lebewesen in Kontakt bringbar ist, wobei ein Latentwärmespeichermittel vorgesehen ist, wobei das Latentwärmespeichermittel derart angeordnet ist, dass zumindest zeitweise durch das Latentwärmespeichermittel eine Temperierung des fließfähigen Materials bewirkbar ist und wobei das Latentwärmespeichermittel als thermochemischer Wärmespeicher ausgebildet ist. Die vorliegende Erfindung bezieht sich ferner auf ein Herstellverfahren zum Herstellen einer Wärmflasche gemäß Anspruch 1 zum zumindest mittelbaren in Kontakt bringen mit einem Lebewesen. Das Herstellverfahren umfasst die in Anspruch 5 offenbarten Schritte. Es ist jedoch ebenfalls denkbar, dass das Herstellungsverfahren bevorzugt mindestens die Schritte des Einbringens eines thermoplastischen Rohlings in eine Blasform, des Formens der Wärmflasche mittels der Einbringung von Luft in den Rohling, des Einsetzens eines Latentwärmespeichermittels in den Innenraum der erzeugten Form durch eine Öffnung der Wärmflasche, wobei das Latentwärmespeichermittel ein Phasenwechselmaterial aufweist, wobei das Phasenwechselmaterial bei einer endothermen Zustandsänderung Energie aufnimmt und bei einer exothermen Zustandsänderung Energie in Form von Wärme abgibt, des Einsetzens eines mit einem Gewinde versehenen Ein- bzw. Auslassmittels in der Öffnung der Wärmflasche und des Verbindens des Ein- bzw. Auslassmittels mit der Wärmflasche aufweist.

Dieses Herstellverfahren ist vorteilhaft, da erstmals ein Weg zur Herstellung einer Wärmflasche mit integriertem Latentwärmespeichermittel bereitgestellt wird.

Weiterhin bezieht sich die vorliegende Erfindung auf ein Latentwärmespeichermittel zur Verwendung als Nachrüstelement für Wärmflaschen gemäß Anspruch 1 bzw. auf ein Wärmflaschen-Latentwärmespeichermittel zur Einbringung in eine zumindest teilweise und bevorzugt größtenteils aus Gummi oder Kunststoff, insbesondere PVC, bestehende Wärmflasche gemäß Anspruch 1, wobei die Wärmflasche eine Öffnung aufweist, die einen Öffnungsdurchmesser von kleiner oder gleich 30 mm, insbesondere kleiner oder gleich 29 mm, insbesondere kleiner oder gleich 28 mm, insbesondere kleiner oder gleich 27 mm, insbesondere kleiner oder gleich 26 mm, insbesondere kleiner oder gleich 25 mm, hat, mindestens 0,4 Liter fließfähiges Material, insbesondere Wasser, insbesondere mindestens 0,5 Liter, insbesondere mindestens 0,75 Liter, insbesondere mindestens 1 Liter, insbesondere mindestens 1,2 Liter, insbesondere mindestens 1,5 Liter, insbesondere mindestens 1,75 Liter, insbesondere mindestens 2 Liter, aufnehmen kann und mindestens eine Wanddicke von 1 mm, insbesondere von mindestens 1,1 mm, insbesondere von mindestens 1,2 mm, insbesondere von mindestens 1,3 mm, insbesondere von mindestens 1,4 mm, insbesondere von mindestens 1,5 mm, aufweist. Das Wärmflaschen-Latentwärmespeichermittel umfasst mindestens eine Wandung, die eine äußere dreidimensionale Form des Wärmflaschen-Latentwärmespeichermittels vorgibt bzw. ausbildet, wobei durch die Wandung ein Vorhalteraum begrenzt wird, in dem sich ein Phasenwechselmaterial befindet, wobei das Phasenwechselmaterial bei einer endothermen Zustandsänderung Energie aufnimmt und bei einer exothermen Zustandsänderung Energie in Form von Wärme abgibt, wobei das Phasenwechselmaterial eine Schmelztemperatur von mehr als 36°C, insbesondere von mehr als 40°C, insbesondere von mehr als 42°C, insbesondere von mehr als 45°C,insbesondere von mehr als 47°C,insbesondere von mehr als 50°C,insbesondere von mehr als 52°C, insbesondere von mehr als 55°C, insbesondere von mehr als 56°C, wie z.B. bis zu 60°C oder bis zu 65°C oder bis zu 70°C, aufweist und in einer Menge bzw. Masse vorgesehen ist, die bevorzugt bei einer 2/3 Füllung, insbesondere bei einer vollen Füllung, der Wärmflasche mit auf über die Schmelztemperatur des Phasenwechselmaterials, insbesondere mit auf 80°C, insbesondere mit auf 95°C erhitztem heißem fließfähigen Material, insbesondere Wasser, bei einer Umgebungstemperatur von 20°C oder von 30°C oder von 36°C oder von 40°C die endotherme Zustandsänderung nur teilweise erfährt. Die Wärmflasche besteht bevorzugt aus einem Polymer- oder Elastomerwerkstoff, insbesondere aus Gummi, PVC oder Latex.

Bevorzugt schließt sich die exotherme Zustandsänderung zeitlich besonders bevorzugt unmittelbar bzw. im Wesentlichen unmittelbar an die endotherme Zustandsänderung an.

Das Wärmflaschen-Latentwärmespeichermittel bzw. das Latentwärmespeichermittel weist bevorzugt eine folienartige Einhausung bzw. Hülle auf, d.h., dass die Wandung des Wärmflaschen-Latentwärmespeichermittel bevorzugt flexibel ausgebildet ist. Besonders bevorzugt besteht die Wandung bzw. Einhausung des Latentwärmespeichermittels bzw. des Wärmflaschen-Latentwärmespeichermittel aus einem Polymerwerkstoff, insbesondere aus einem flüssigkeitsdichten, insbesondere zumindest bei Umgebungsdruck, und temperaturstabilen, insbesondere bis mindestens 100°C, bevorzugt bis oder im Wesentlichen bis 110 °C, bevorzugt bis oder im Wesentlichen bis 120 °C, bevorzugt bis oder im Wesentlichen bis 130 °C, bevorzugt bis oder im Wesentlichen bis 140 °C, bevorzugt bis oder im Wesentlichen bis 150 °C, bevorzugt bis oder im Wesentlichen bis 160 °C, bevorzugt bis oder im Wesentlichen bis 170 °C, bevorzugt bis oder im Wesentlichen bis 200 °C, bevorzugt bis oder im Wesentlichen bis 250 °C, bevorzugt bis oder im Wesentlichen bis 300 °C, bevorzugt bis oder im Wesentlichen bis 350 °C, Polymerwerkstoff.

Das Wärmflaschen-Latentwärmespeichermittel kann bevorzugt ebenfalls aus einer Vielzahl einzelner voneinander körperlich getrennter oder miteinander verbundener jeweils mit einem Phasenwechselmaterial gefüllten und bevorzugt folienartig eingehausten Aufnahmeräumen gebildet werden. Es ist hierbei bevorzugt, dass das gesamte Phasenwechselmaterial durch die mittels dem heißen fließfähigen Material, insbesondere Wasser, erfolgende Erwärmung nicht infolge der endothermen Reaktion und einem dadurch bewirkten vollständigen Zustandswechsel in einen stabilen Zustand überführt wird, sondern bevorzugt stets selbsttätig mittels einer exothermen Reaktion den Ausgangszustand, der vor der Erhitzung vorgelegen hat, wieder annimmt. Sollte das Phasenwechselmaterial nämlich durch die endotherme Reaktion vollständig einen Zustandswechsel erfahren, dann könnte das Phasenwechselmaterial nicht ohne eine externe Auslösung zum exothermen Zustandswechsel veranlasst werden. Das Wärmflaschen-Latentwärmespeichermittel muss somit bevorzugt derart gestaltet sein, dass es in einem Ausgangszustand, in dem die endotherme Reaktion möglich bzw. noch möglich ist, in eine Wärmflasche einbringbar ist. Da ein Phasenwechselmaterial in diesem Zustand in der Regel eine Festkörperform annimmt sind für die Einbringung in die Wärmflasche bestimmte geometrische Voraussetzungen erforderlich, insbesondere muss das Wärmflaschen-Latentwärmespeichermittel durch die Öffnung der Wärmflasche passen. Durch die begrenzte Öffnungsweise einer Wärmflasche muss das Wärmflaschen-Latentwärmespeichermittel jedoch eine Mindestlänge bzw. Mindestmenge an Phasenwechselmaterial aufweisen, um durch das heiße fließfähige Material, insbesondere Wasser, nicht vollständig infolge einer endothermen Reaktion in den angesprochenen stabilen Zustand überführt zu werden. Zum Beispiel liegt ein Wärmflaschen-Latentwärmespeichermittel vor, das als Phasenwechselmaterial Natriumacetat aufweist. Das Phasenwechselmaterial muss vor der Einbringung in die Wärmflasche zumindest teilweise in einer kristallisierten Form vorliegen, damit die exotherme Reaktion entweder läuft oder abgeschlossen ist. Sollte das Phasenwechselmaterial im vollständig flüssigen Zustand vorliegen, d.h. den endothermen Zustandswechsel vollständig umgesetzt haben, dann könnte eine Auslösung des exothermen Zustandswechsels nicht mehr bewirkt werden, wenn das Wärmflaschen-Latentwärmespeichermittel in die Wärmflasche eingebaut wurde. Das Wärmflaschen-Latentwärmespeichermittel mit dem zumindest teilweise und bevorzugt vollständig kristallisierten Phasenwechselmaterial wird durch die Öffnung der Wärmflasche in die Wärmflasche eingebracht. Infolge der Heißwasserzuführung bzw. der Zuführung des temperierten fließfähigen Materials in die Wärmflasche schmilzt das Phasenwechselmaterial unter Aufnahme von Wärme (Schmelzwärme) teilweise. Es ist hierbei wesentlich, dass das Phasenwechselmaterial nicht vollständig schmilzt und daher das Latentwärmespeichermittel die entsprechende Form aufweist, insbesondere eine entsprechende Dicke aufweist, und/oder das Phasenwechselmaterial in der entsprechenden Menge bzw. Masse vorgesehen ist. Durch die Aufnahme der Schmelzwärme wird die Temperatur des zugeführten fließfähige Materials, insbesondere Wassers, abgekühlt. Fällt die Temperatur des zugeführten fließfähigen Materials, insbesondere Wassers, unter eine bestimmte Schwellentemperatur, insbesondere die Erstarrungstemperatur des Phasenwechselmaterials, dann beginnt das Phasenwechselmaterial selbsttätig zu erstarren und gibt dabei Wärme an das fließfähige Material, insbesondere Wasser, bzw. die Wärmflaschenwandung ab.

Weiterhin erstreckt sich das Gewinde der Wärmflasche in axialer Richtung bevorzugt mindestens 3mm und besonders bevorzugt mindestens 4 mm, 5 mm, 6 mm, 7 mm, 8 mm, 9 mm, 10 mm oder mehr als 10 mm. Das Wärmflaschen-Latentwärmespeichermittel ist bevorzugt ferner derart ausgebildet, dass es ohne die Zuhilfenahme eines Werkzeugs oder ohne dabei zerstört zu werden nicht aus der Wärmflasche entnehmbar ist.

Das Wärmflaschen-Latentwärmespeichermittel ist besonders bevorzugt als Nachrüstmittel zum Nachrüsten von Wärmflaschen geeignet. Bevorzugt ist das Wärmflaschen-Latentwärmespeichermittel schlauchförmig ausgebildet. Das schlauchförmig ausgebildete Wärmflaschen-Latentwärmespeichermittel weist bevorzugt abschnittsweise in einem Querschnitt orthogonal zur Längserstreckungsrichtung des schlauchförmig ausgebildeten Wärmflaschen-Latentwärmespeichermittel eine insbesondere runde Form auf. Der Durchmesser des Wärmflaschen-Latentwärmespeichermittel ist bevorzugt kleiner oder gleich 30 mm, insbesondere kleiner oder gleich 29 mm, insbesondere kleiner oder gleich 28 mm, insbesondere kleiner oder gleich 27 mm, insbesondere kleiner oder gleich 26 mm, insbesondere kleiner oder gleich 25 mm, insbesondere kleiner oder gleich 24 mm, insbesondere kleiner oder gleich 23 mm, insbesondere kleiner oder gleich 22 mm, insbesondere kleiner oder gleich 21 mm, insbesondere kleiner oder gleich 20 mm, Das schlauchförmig ausgebildete Wärmflaschen-Latentwärmespeichermittel erstreckt sich in seiner axialen Richtung bzw. in seiner Längsrichtung bevorzugt genau, mindestens oder maximal 50 mm, genau, mindestens oder maximal 60 mm, genau, mindestens oder maximal 70 mm, genau, mindestens oder maximal 80 mm, genau, mindestens oder maximal 90 mm, genau, mindestens oder maximal 100 mm, genau, mindestens oder maximal 110 mm, genau, mindestens oder maximal 120 mm, genau, mindestens oder maximal 130 mm, genau, mindestens oder maximal 140 mm, genau, mindestens oder maximal 150 mm, genau, mindestens oder maximal 160 mm, genau, mindestens oder maximal 170 mm, genau, mindestens oder maximal 180 mm, genau, mindestens oder maximal 190 mm, genau, mindestens oder maximal 200 mm, genau, mindestens oder maximal 210 mm, genau, mindestens oder maximal 250 mm, genau, mindestens oder maximal 300 mm, genau, mindestens oder maximal 3500 mm, genau, mindestens oder maximal 4000 mm.

Die Erfindung kann sich ebenfalls auf eine Wärmflasche mit mindestens einem Wärmflaschen-Latentwärmespeichermittel beziehen.

Im Sinne der vorliegender Erfindung kann als fließfähiges Material eine Flüssigkeit verstanden werden. Ein bevorzugtes Fluid ist hierbei Wasser, wobei alternativ auch Öl oder sonstige Flüssigkeiten oder Gele oder Fette oder Creme denkbar währen. Weiterhin sind als nicht beanspruchtes Schüttgut bevorzugt Kerne oder Samen zu verstehen. Kerne können hierbei z.B. Kirschkern, Traubenkerne, Kernmischungen, etc. sein. Samen können hierbei z.B. Leinsamen sein. Weiterhin werden im Sinne eines nicht beanspruchten Beispiels ebenfalls Sand, Erde, Steine, Moor, etc. als fließfähige Materialien angesehen.

Die vorliegende Erfindung bezieht sich ferner auf ein Set aus einer zuvor beschrieben Wärmeeinrichtung und einer austauschbaren Ummantelung, insbesondere einer textilen Ummantelung, wobei die Ummantelung zumindest die flexible Wandung der Wärmeinrichtung abschnittsweise umschließt. Besonders bevorzugt weist das Set einen Verschluss zum Öffnen und Verschließen der Wärmeeinrichtung mit auf. Die Ummantelung besteht bevorzugt aus einem Material ausgewählt aus der Gruppe zumindest bestehend aus Fleece, Polymermaterial, wie z.B. Neopren, Baumwolle, Wolle, Frottee, etc. Besonders bevorzugt bildet die Ummantelung zwei voneinander unterschiedlich stark wärmeisolierende Anteile aus, wobei die unterschiedlich stark wärmeisolierenden Anteile durch die Wärmeeinrichtung voneinander beabstandet werden, wenn die Ummantelung die Wärmeeinrichtung umschließt. Die Ummantelung ist dabei in Form einer Hülle ausgebildet, in welche die Wärmeeinrichtung einbringbar ist. Es ist hierbei denkbar, dass ein Anteil der Ummantelung dünner ausgebildet ist als der andere Anteil der Ummantelung. Die Anteile der Ummantelung sind derart gestaltet, dass sie an gegenüberliegenden und voneinander beabstandeten Wandungsteilen der Wärmeeinrichtung anliegen und diese bevorzugt jeweils vollständig abdecken oder überlagern. Es hat sich gezeigt, dass die Effekte der erfindungsgemäßen Wärmeeinrichtung, insbesondere Wärmflasche, durch die Verwendung einer Ummantelung noch stärker hervortreten.

Die Verwendung der Wörter "im Wesentlichen" definiert bevorzugt in allen Fällen, in denen diese Wörter im Rahmen der vorliegenden Erfindung verwendet werden eine Abweichung im Bereich von 1%-30%, insbesondere von 1%-20%, insbesondere von 1%-10%, insbesondere von 1%-5%, insbesondere von 1%-2%, von der Festlegung, die ohne die Verwendung dieser Wörter gegeben wäre. Einzelne oder alle Darstellungen der im Nachfolgenden beschriebenen Figuren sind bevorzugt als Konstruktionszeichnungen anzusehen, d.h. die sich aus der bzw. den Figuren ergebenden Abmessungen, Proportionen, Funktionszusammenhänge und/oder Anordnungen entsprechen bevorzugt genau oder bevorzugt im Wesentlichen denen der erfindungsgemäßen Vorrichtung bzw. des erfindungsgemäßen Produkts. Weitere Vorteile, Ziele und Eigenschaften vorliegender Erfindung werden anhand nachfolgender Beschreibung anliegender Zeichnungen erläutert, in welchen beispielhaft erfindungsgemäße Wärmeeinrichtungen dargestellt sind. Elemente der erfindungsgemäßen Einrichtungen und Verfahren, welche in den Figuren wenigsten im Wesentlichen hinsichtlich ihrer Funktion übereinstimmen, können hierbei mit gleichen Bezugszeichen gekennzeichnet sein, wobei diese Bauteile bzw. Elemente nicht in allen Figuren beziffert oder erläutert sein müssen. Nachfolgend wird die Erfindung rein beispielhaft anhand der beigefügten Figuren beschrieben.

Darin zeigen:
- Fig. 1: eine Schnittdarstellung einer Wärmeeinrichtung gemäß einer bevorzugte Ausführungsform der vorliegenden Erfindung;
- Fig. 2a: eine Schnittdarstellung einer Wärmeeinrichtung gemäß einer weiteren bevorzugte Ausführungsform der vorliegenden Erfindung;
- Fig. 2b und 2c: Schnittdarstellungen verschiedener Wärmeeinrichtung gemäß einer bevorzugte Ausführungsform der vorliegenden Erfindung, wobei die einzelnen Wärmeeinrichtungen unterschiedliche Latentwärmespeichermittel bzw. unterschiedliche Anzahlen an Latentwärmespeichermittel aufweisen;
- Fig. 3a und 3b: Schnittdarstellungen verschiedener Wärmeeinrichtung gemäß einer bevorzugte Ausführungsform der vorliegenden Erfindung,
- Fig. 4a: eine Schnittdarstellung einer Wärmeeinrichtung gemäß einer weiteren bevorzugte Ausführungsform der vorliegenden Erfindung;
- Fig. 4b: eine Draufsicht auf die in Fig. 4a gezeigte Wärmeeinrichtung;
- Fig. 4c: eine Schnittdarstellung einer Wärmeeinrichtung gemäß einer weiteren bevorzugte Ausführungsform der vorliegenden Erfindung;
- Fig. 4d: eine Draufsicht auf die in Fig. 4c gezeigte Wärmeeinrichtung;
- Fig. 5a und 5b: Schnittdarstellungen verschiedener Wärmeeinrichtung gemäß einer weiteren bevorzugte Ausführungsform der vorliegenden Erfindung,
- Fig. 5c: eine Draufsicht auf die in den Figuren 5a und 5b gezeigte Wärmeeinrichtung;
- Fig. 5d: eine rückseitige Ansicht der in den Figuren 5a bis 5c gezeigten Wärmeeinrichtungen,
- Fig. 6: eine Draufsicht auf eine zumindest teilweise transparente Wärmeeinrichtung gemäß einer weiteren bevorzugten Ausführungsform der vorliegenden Erfindung,
- Fig. 7: eine gewöhnliche Abkühlkurve und eine Abkühlkurve im Sinne der vorliegenden Erfindung,
- Fig. 8a und 8b: eine weitere bevorzugte Ausführungsform der vorliegenden Erfindung, gemäß der das Latentwärmespeichermittel bevorzugt an einer den Aufnahmeraum begrenzenden Wandung fixiert ist,
- Fig. 9a und 9b: jeweils eine weitere bevorzugte Ausführungsform der vorliegenden Erfindung, wobei das Latentwärmespeichermittel in beiden Darstellungen eine sehr große Oberfläche aufweist,
- Fig. 10: ein Diagramm, dem Abkühlkurven verschiedene Wärmflaschenkonfigurationen entnehmbar sind, und
- Fig. 11a, 11b, 11c: drei verschiede Ansichten eines exemplarischen Latentwärmespeichermittels, insbesondere für die Verwendung in einer Wärmflasche.

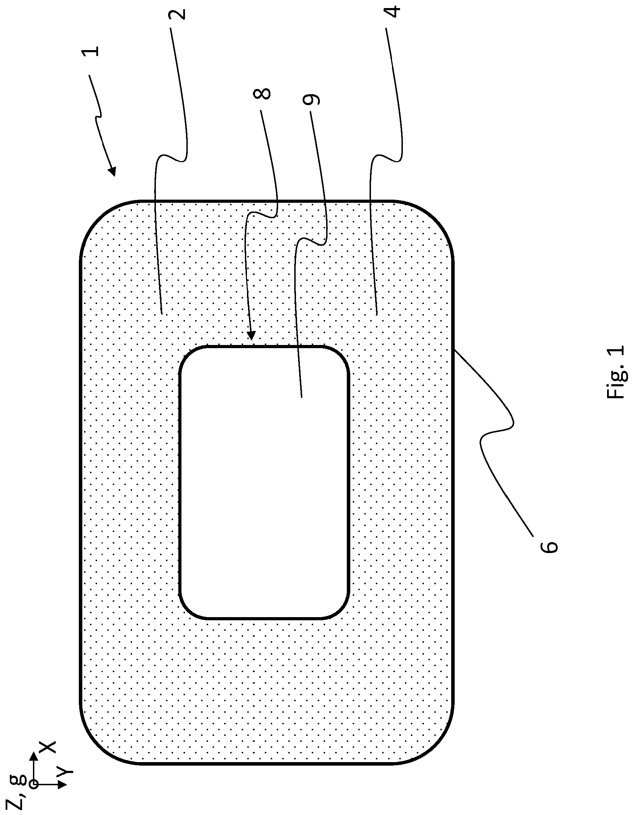

Fig. 1 zeigt eine erfindungsgemäße Wärmeeinrichtung 1. Die Wärmeeinrichtung 1 ist bevorzugt als Wärmekissen ausgebildet. Die Wärmeeinrichtung 1 weist einen Aufnahmeraum 2 auf, der durch eine Wandung 6 ausgebildet bzw. begrenzt wird. Die Wandung 6 kann hierbei aus Gummi, einem Polymer, insbesondere PVC, oder aus einem textilen Werkstoff, insbesondere einem Gewirk, hergestellt sein. Der Aufnahmeraum 2 ist mit einem fließfähigen Material 4 zumindest teilweise gefüllt. Das fließfähige Material 4 ist bevorzugt ein Schüttgut, wie z.B. Sand, Kerne, Steine, oder ein Fluid, wie z.B. Wasser, Moor oder ein Gel, etc.. Es erschließt sich hierbei, dass die Wandung der Wärmeeinrichtung 1 derart gewählt bzw. ausgebildet ist, dass sie das jeweilige fließfähige Material 4 im Wesentlichen verlustfrei bzw. verlustfrei vorhalten kann. Im Aufnahmeraum 2 ist neben dem fließfähigen Material 4 bzw. umgeben von dem fließfähigen Material 4 mindestens ein Latentwärmespeichermittel 8 vorgesehen. Das mindestens eine Latentwärmespeichermittel 8 kann hierbei lose in dem Aufnahmeraum 2 vorgesehen bzw. angeordnet sein oder es kann mittels einer Befestigung gegenüber der Wandung 6 positioniert sein bzw. an der Wandung 6 angeordnet bzw. fixiert sein. Das Bezugszeichen 9 kennzeichnet schematisch den Innenraum des Latentwärmespeichermittels, der durch eine bevorzugt folienartige Wandung begrenzt wird. In dem Innenraum 9 des Latentwärmespeichermittels ist ein Phasenwechselmaterial angeordnet.

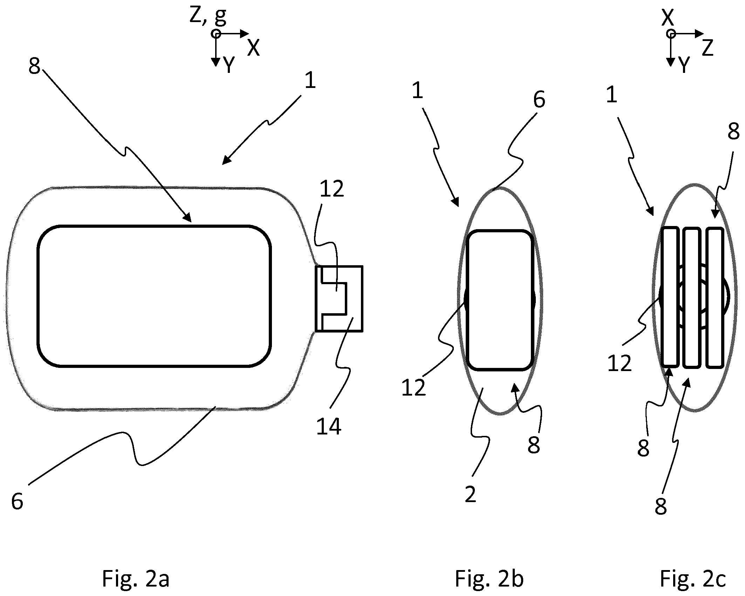

In Fig. 2a ist eine Schnittdarstellung einer bevorzugt als Wärmflasche ausgebildeten Wärmeeinrichtung 1 dargestellt. Die Wärmeeinrichtung 1 weist hierbei mindestens oder genau eine Zuführ- und Entleeröffnung 12 auf. Die Zuführ- und Entleeröffnung 12 ist hierbei bevorzugt mittels eines Verschluss 14 verschließbar. Der Verschluss 14 kann hierbei je nach Ausgestaltung der Zuführ- und Entleeröffnung 12 mit einem Außengewinde oder einem Innengewinde oder mittels eines beliebigen anderen abdichtenden Verschlusskonzepts ausgebildet sein.

In den Figuren 2b und 2c sind Schnittdarstellungen gezeigt, wobei die Schnitte in der Y-Z-Ebene geführt sind. Der Darstellung 2b lässt sich ein Latentwärmespeichermittel 8 innerhalb des Aufnahmeraums 2 entnehmen. Die Darstellung 2c zeigt, dass innerhalb des Aufnahmeraums 2 mehrere Latentwärmespeichermittel 8 angeordnet sind. Es lässt sich hierdurch ableiten, dass die in der Fig. 2c gezeigten Latentwärmespeichermittel 8 zusammen eine größere Oberfläche aufweisen als das in Fig. 2b gezeigte Latentwärmespeichermittel 8, wobei das in Fig. 2b gezeigte Latentwärmespeichermittel 8 bei einer gleich langen Erstreckung in X-Richtung bzw. in der Längsrichtung der Wärmeeinrichtung 1 eine größere Menge an Phasenwechselmaterial aufweisen kann bzw. aufweist.

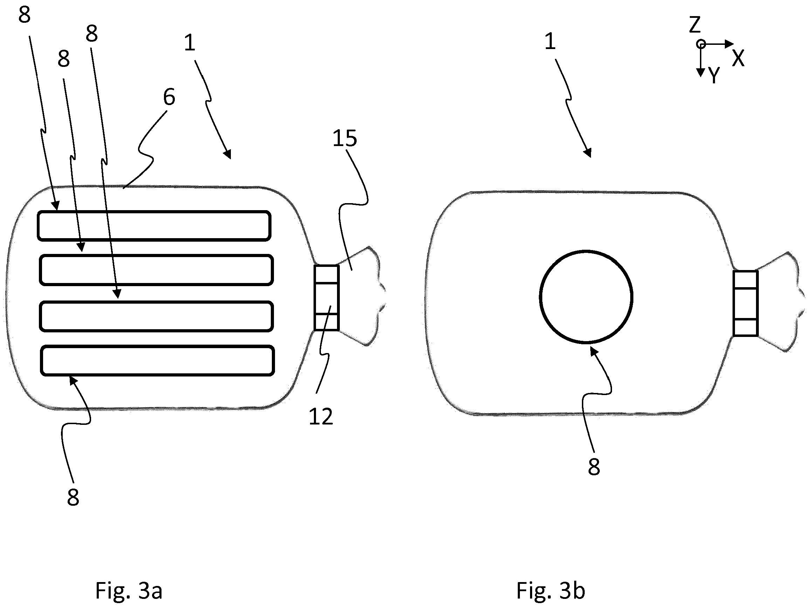

In den in Fig. 3a und 3b gezeigten Darstellungen weisen die Wärmeeinrichtungen 1 jeweils einen Trichter 15 zum vereinfachten Einfüllen einer fließfähigen Substanz auf. Es ist hierbei jedoch denkbar, dass die dargestellten Wärmeeinrichtungen 1 auch ohne einen solchen Trichter 15 ausgebildet werden. Ferner ist denkbar, dass alle in den weiteren Figuren gezeigten Wärmeeinrichtungen 1, die eine Zuführ- und Entleeröffnung 12 bzw. ein Ein- und Auslassmittel 12 aufweisen, einen solchen Trichter 15 aufweisen können. Ferner sind die einzelnen dargestellten Zuführ- und Entleeröffnungen 12, die in unterschiedlichen Ausführungen dargestellt sind als rein exemplarisch anzusehen.

Der Fig. 3a lässt sich ferner entnehmen, dass innerhalb des Aufnahmeraums 2 mehrere Latentwärmespeichermittel 8 vorgesehen sind. Es ist somit denkbar, dass innerhalb eine Aufnahmeraums 2 mindestens oder genau zwei, mindestens oder genau drei, mindestens oder genau vier, mindestens oder genau fünf, mindestens oder genau sechs, mindestens oder genau sieben Latentwärmespeichermittel 8 vorsehbar bzw. einbringbar bzw. anordenbar sind.

In Fig. 3b ist eine Ausführungsform gezeigt, gemäß der das Latentwärmespeichermittel 8 sphärisch bzw. im Wesentlichen sphärisch, insbesondere scheibenförmig oder kugelförmig, ausgebildet ist.

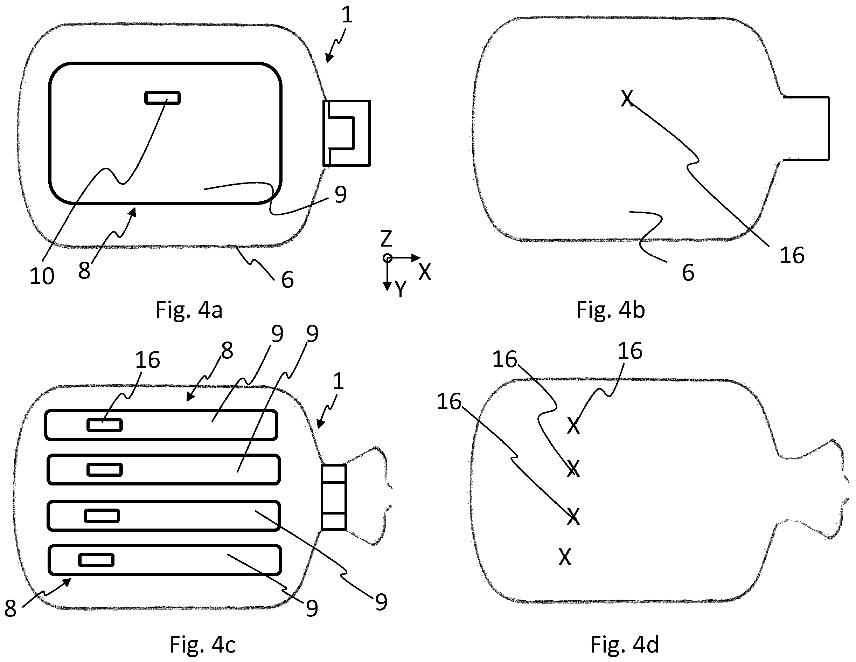

In Fig. 4a ist eine Wärmeeinrichtung 1 gezeigt, die bevorzugt als Wärmflasche ausgeführt ist. Die Wärmeeinrichtung 1 weist hierbei mindestens ein Latentwärmespeichermittel 8 auf. Das Latentwärmespeichermittel 8 weist ein Betätigungsmittel 10, insbesondere eine Metallfeder bzw. einen Knackfrosch, zum Auslösen der exothermen Zustandsänderung des Phasenwechselmaterials auf. Bevorzugt ist in dieser Ausführungsform das Betätigungsmittel 10 und/oder das Latentwärmespeichermittel 8 stets definiert gegenüber einem Wandungsteil 6 der Wärmeeinrichtung 1, insbesondere gegenüber einer an dem Wandungsteil 6 angebrachten oder vorgesehenen Markierung 16 (vgl. Fig. 4b), angeordnet.

Es ist ebenfalls denkbar, dass ein bzw. mindestens ein Latentwärmespeichermittel 8 mit einem Betätigungsmittel 10 innerhalb einer als Wärmekissen ausgebildeten Wärmeeinrichtung 1 vorgesehen ist. Bevorzugt ist das Betätigungsmittel 10 in diesem Falle ebenfalls definiert gegenüber einem Wandungsteil 6 der Wärmeeinrichtung 1 angeordnet.

In Fig. 4b ist eine Draufsicht auf eine Wärmeeinrichtung 1 gezeigt. Es ist dieser Darstellung zu entnehmen, dass auf der Wandung 6 eine Markierung 16 vorgesehen ist. Die Markierung 16 zeigt hierbei bevorzugt die Position bzw. im Wesentlichen die Position des Betätigungselements 10 an.

In den Figuren 4c und 4d ist ein zu den Figuren 4a und 4b analoges Beispiel gezeigt, wobei in den Figuren 4c und 4d mehr Latentwärmespeichermittel 8 und daher mehr Markierungen 16 gezeigt sind. Es ist hierbei bevorzugt denkbar, dass die Anzahl der Markierungen 16 mit der Anzahl der Betätigungselemente 10 korreliert.

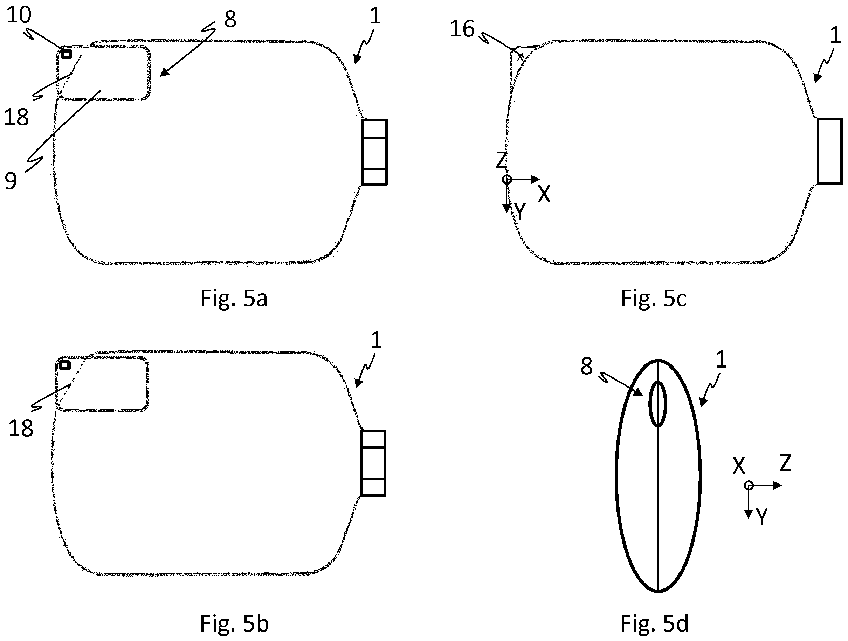

In der Fig. 5a ist eine Ausführungsform in einer Schnittdarstellung gezeigt, gemäß der ein Teil des Latentwärmespeichermittels 8 außerhalb des Aufnahmeraums 2 angeordnet ist. In dem außerhalb des Aufnahmeraums 2 angeordneten Bereichs des Latentwärmespeichermittels 8 ist bevorzugt ein Betätigungsmittel 10 angeordnet. Bevorzugt ist der außerhalb des Aufnahmeraums 2 ausgebildete Anteil des Latentwärmespeichermittels 8 derart gegenüber dem innerhalb des Aufnahmeraums 2 angeordneten Anteils des Latentwärmespeichermittels 8 abgegrenzt, dass das Betätigungsmittel 10 sich bevorzugt ausschließlich außerhalb des Aufnahmeraums 2 befindet. Bevorzugt ist zumindest der Teil des Latentwärmespeichermittels 8, in dem das Betätigungsmittel 10 angeordnet ist, teilweise oder vollständig transparent ausgebildet. Weiterhin ist der außerhalb des Aufnahmeraums 2 angeordnete Anteil des Latentwärmespeichermittels 8 derart vom sich innerhalb des Aufnahmeraums 2 befindenden Anteils des Latentwärmespeichermittels 8 abgegrenzt, dass die exotherme Zustandsänderung und die endotherme Zustandsänderung des im Innenraum 9 des Latentwärmespeichermittels 8 vorgehaltenen Phasenwechselmaterials bevorzugt vollständig erfolgen können. Weiterhin kennzeichnet das Bezugszeichen 18 eine Abgrenzung, die wie in Fig. 5a gezeigt z.B. eine Lücke aufweisen kann oder - wie in Fig. 5b gezeigt - z.B. durch eine Art Perforation gebildet werden kann.

Weiterhin ist denkbar, dass eine Wärmeeinrichtung 1 mehrere Latentwärmespeichermittel 8 aufweisen kann, die derart angeordnet sind, dass sie bei geschlossener Wärmeeinrichtung 1 von einem Nutzer berührbar sind. Bevorzugt weist eine insbesondere als Wärmflasche ausgebildete Wärmeeinrichtung 1 mehrere, insbesondere mindestens, genau oder maximal 2, mindestens, genau oder maximal 3, mindestens, genau oder maximal 4, mindestens, genau oder maximal 5, mindestens, genau oder maximal 6 Latentwärmespeichermittel 8 auf, die bei geschlossener Wärmeeinrichtung 1 von einem Nutzer berührbar sind oder die sich aus dem Aufnahmeraum 2 heraus erstrecken.

Fig. 5c zeigt eine Draufsicht auf eine erfindungsgemäße Wärmeeinrichtung 1, wobei bevorzugt im Bereich des Latentwärmespeichermittels 8 oder bevorzugt an dem Latentwärmespeichermittel 8 eine Markierung 16 zum Anzeigen der Position des Betätigungsmittels 10 vorgesehen ist. Es ist jedoch ebenfalls denkbar, dass keine Markierung 16 erforderlich ist, da durch die räumliche Abgrenzung des Bereichs, in dem sich das Betätigungsmittel 10 befindet, direkt ersichtlich wird, wo das Betätigungsmittel 10angeordnet ist. Ferner ist denkbar, dass das Latentwärmespeichermittel 8 zumindest teilweise oder lokal derart transparent ausgebildet ist, dass erkennbar ist, wo sich das Betätigungsmittel 10 befindet.

Fig. 5d zeigt eine rückseitige Ansicht der in den Figuren 5a-5c gezeigten Wärmeeinrichtung 1. Das Bezugszeichen 20 kennzeichnet hierbei eine sich bei der Herstellung der Wärmeeinrichtung, insbesondere durch eine Blasform oder beim Vulkanisieren, ergebende und bevorzugt umlaufende Naht.

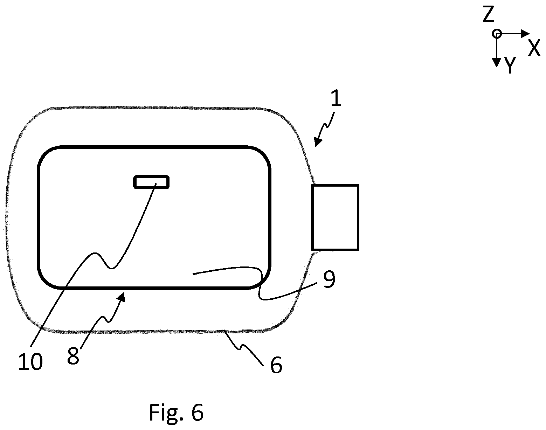

Fig. 6 zeigt eine Draufsicht auf eine zumindest einerseits und bevorzugt beiderseits zumindest teilweise und bevorzugt im Wesentlichen oder besonders bevorzugt vollständig transparente Wärmeeinrichtung 1. Aufgrund der Transparenz der Wandung ist ersichtlich, wo sich das Latentwärmespeichermittel 8 und das darin angeordnete Betätigungsmittel 10 befinden. Das Latentwärmespeichermittel 8 ist bevorzugt ebenfalls zumindest einerseits und bevorzugt beiderseits zumindest teilweise und bevorzugt im Wesentlichen oder besonders bevorzugt vollständig transparent ausgebildet.

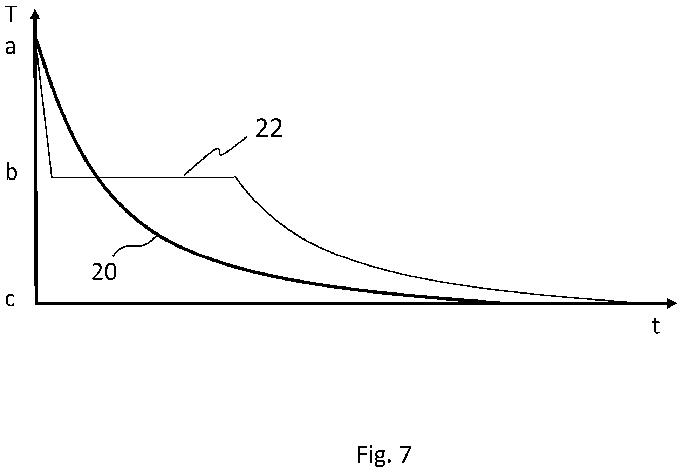

In Fig. 7 sind zwei verschiedene schematische Temperaturverläufe 20 und 22 gezeigt. Der Temperaturverlauf 20 zeigt im Wesentlichen eine Abkühlung einer aus dem Stand der Technik bekannten Wärmeeinrichtung und der Temperaturverlauf 22 zeigt im Wesentlichen eine Abkühlung einer erfindungsgemäßen Wärmeeinrichtung 1. Gemäß den beiden Kurven wird die Wärmeeinrichtung 1 auf eine Starttemperatur a, insbesondere eine Temperatur zwischen 60°C und 100°C und bevorzugt von 95°C bzw. von im Wesentlichen 95°C, temperiert. Die Temperierung kann hierbei durch das Einfüllen eines fließfähigen Materials 4, insbesondere Wasser, in die Wärmeeinrichtung 1 erfolgen. Gegensätzlich zum gewöhnlichen Temperaturverlauf 20 kühlt das fließfähige Material gemäß der vorliegenden Erfindung zunächst schneller ab. Diese schnellere Abkühlung wird durch die Übertragung von Wärme vom fließfähigen Material 4 auf das Latentwärmespeichermittel 8, welches sich innerhalb des Aufnahmeraums 2 oder an dem Aufnahmeraum 2 oder in der Wandung 6 des Aufnahmeraums 2 der Wärmeeinrichtung 1 befindet, bewirkt. Durch die Wärmezuführung erfolgt innerhalb des Latentwärmespeichermittels 8 teilweise ein endothermer Zustandswechsel des sich darin befindenden Phasenwechselmaterials. Wenn das fließfähige Material auf eine bzw. unterhalb eine Schwellentemperatur des Phasenwechselmaterials abkühlt, erfolgt eine Rückgängigmachung des endothermen Zustandswechsels durch einen exothermen Zustandswechsel. Das Phasenwechselmaterial gibt dann solange die beim endothermen Zustandswechsel aufgenommene Energie in Form von Wärme ab, bis es wieder vollständig im Ausgangszustand, insbesondere als Festkörper, vorliegt. Durch die Wärmeabgabe des Phasenwechselmaterials erfolgt eine Modifizierung der Abkühlkurve bzw. eine Temperierung des fließfähigen Materials, wodurch dieses für einen bestimmten Zeitraum bevorzugt auf einer konstanten bzw. im Wesentlichen konstanten Temperatur gehalten wird. Beide Kurven 20, 22 kühlen bis auf die Umgebungstemperatur c, insbesondere auf 20°C, ab. Das Latentwärmespeichermittel 8 ist somit bevorzugt derart ausgebildet, dass bei einer definierten Menge, insbesondere einer vollen Füllung des Aufnahmeraums 2, an definiert temperiertem, insbesondere mit 95°C eingefülltem, fließfähigem Material, insbesondere Wasser, die endotherme Zustandsänderung des Phasenwechselmaterials nur teilweise bewirkbar ist, insbesondere damit die Zustandsänderung selbsttätig in den Ausgangszustand, vor der Zustandsänderung, zurückversetzt wird.

Der gemäß Fig. 7 beschriebene Temperaturverlauf tritt besonders bevorzugt bei den in den Fig. 2a-2c und 3a-3b gezeigten Ausführungsformen auf.

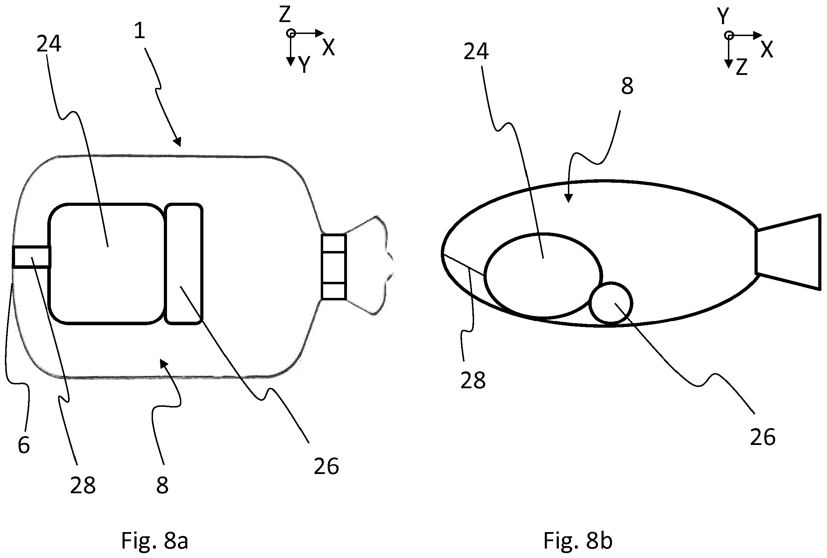

In Fig. 8a ist eine Querschnittsdarstellung einer erfindungsgemäßen Wärmeinrichtung 1 mit einem modifizierten Latentwärmespeichermittel 8 gezeigt. Das Wärmflaschen-Latentwärmespeichermittel bzw. Latentwärmespeichermittel 8 weist hierbei mehrere Kammern 24 und 26 auf, in denen sich Phasenwechselmaterial befindet. Die Kammern 24, 26 sind derart miteinander funktional bzw. körperlich bzw. kommunizierend verbunden, dass ein in der Hauptkammer 24 ausgelöster exothermer Zustandswechsel auf die andere/n Kammer/n, d.h. die Nebenkammer/n 26, übergeht. Diese Ausgestaltung hat den Vorteil, dass eine oder mehrere Nebenkammern 26 vorgesehen werden können, um z.B. kühlrippenartig eine möglichst große Wärmeübertragungsfläche auszubilden. Die Hauptkammer 24 ist dabei jedoch bevorzugt derart ausgebildet, dass das in ihr vorgehaltene Phasenwechselmaterial bei einem maximalen Wärmeeintrag mittels zugeführtem temperierten fließfähigen Material nur teilweise zu einem endothermen Zustandswechsel führt. Das in der bzw. den Nebenkammern vorgehaltene Phasenwechselmaterial kann jedoch vollständig einen Zustandswechsel ausführen. Die Nebenkammer/n 26 weisen bevorzugt ein von der Hauptkammer 24 verschiedenes "Oberfläche zu Phasenwechselmaterialmenge"-Verhältnis auf, wobei die Hauptkammer 24 bevorzugt bei der gleichen Menge an Phasenwechselmaterial eine kleinere Oberfläche aufweist. Es ist jedoch denkbar, dass die Gesamtheit aller Nebenkammern 26 mehr Phasenwechselmaterial und insgesamt eine größere Oberfläche als die Hauptkammer 24 aufweisen.

Das Bezugszeichen 28 kennzeichnet ein bevorzugt flexibles Fixierungselement. Das Fixierungselement 28 dient bevorzugt zum Anordnen des Latentwärmespeichermittels 8 an der Wandung 6 der Wärmeeinrichtung 1. Bevorzugt weist das Fixierungselement 28 einen Kunststoff auf. Besonders bevorzugt ist das Fixierungselement 28 ein Bestandteil des Latentwärmespeichermittels 8. Es wird hierbei ausdrücklich darauf hingewiesen, dass das Fixierungselement 28 nur exemplarisch an einem Latentwärmespeichermittel 8 ausgebildet ist, das eine Nebenkammer 26 aufweist. Es ist ebenfalls denkbar, dass es an einem Latentwärmespeichermittel 8 angeordnet oder ausgebildet ist, das keine oder mehre Nebenkammern 26 aufweist. Bevorzugt wird das Fixierungselement 28 beim Herstellen der bevorzugt als Wärmflasche ausgebildeten Wärmeeinrichtung 1 mit der Wandung 6 der Wärmeeinrichtung 1 verbunden. Das Fixierungselement 28 ist vorteilhaft, da es bevorzugt das Verstopfen des Auslasses durch das Latentwärmespeichermittel 8 beim Auskippen des in der bevorzugt als Wärmflasche ausgebildeten Wärmeinrichtung 1 vorgehaltenen fließfähigen Materials verhindert.

In Figur 8b ist rein beispielhaft eine aus einer Seitenansicht gezeigte Schnittdarstellung der in Fig. 8a gezeigten Wärmeeinrichtung 1 gezeigt.

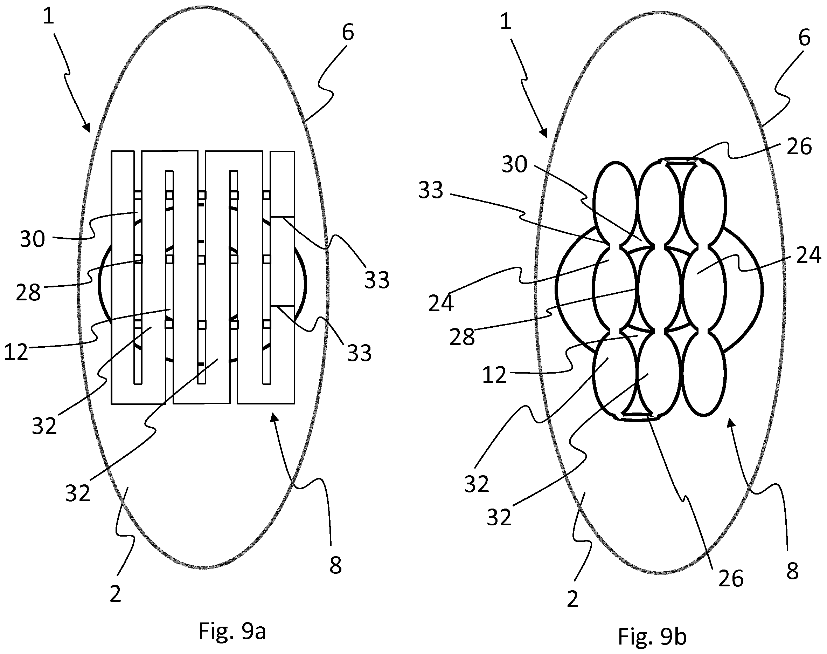

In Fig. 9a ist eine Querschnittsansicht einer Wärmeeinrichtung 1 gezeigt. Das gezeigte Latentwärmespeichermittel 8 kann sich dabei in einer anderen Ebene z.B. entsprechend dem in der Fig. 2a oder dem in der Fig. 6 gezeigten Latentwärmespeichermittel 8 erstrecken. Das Latentwärmespeichermittel 8 ist dadurch gekennzeichnet, dass seine Oberfläche gegenüber seinem Volumen relativ groß ist. Bevorzugt weist das Latentwärmespeichermittel 8 zwei oder mehr, insbesondere drei oder mehr, vier oder mehr, 5 oder mehr sich gegenseitig überlagernde und jeweils zumindest teilweise sich in zueinander parallelen Ebenen erstreckende Vorhaltebereiche, in denen ein Phasenwechselmaterial vorgesehen ist, auf. Bevorzugt sind mindestens zwei und bevorzugt mindestens drei und besonders bevorzugt mindestens 4, 5, 6, oder mehr oder alle sich gegenseitig überlagernde Vorhaltebereiche kommunizierend miteinander verbunden. Kommunizierend miteinander verbunden bedeutet hierbei bevorzugt, dass eine in einem Vorhaltebereich erfolgende Kristallisation bzw. Erstarrung bzw. Verfestigung bzw. ein Phasenwechsel (von flüssig zu fest) auf einen anderen Vorhaltebereich 32 übergehen kann bzw. übergeht. Besonders bevorzugt bedeutet kommunizierend miteinander verbunden, dass das Phasenwechselmaterial im flüssigen Zustand teilweise aus einem Vorhaltebereich 32 in einen anderen Vorhaltebereich 32 überführbar bzw. leitbar ist. Die Wandung des Latentwärmespeichermittels 8 ist somit bevorzugt die Wandung der Vorhaltebereiche 32. Bevorzugt ist die Wandung des Latentwärmespeichermittels 8 flexibel, bevorzugt besteht die Wandung aus einem Polymer. Besonders bevorzugt weist das Latentwärmespeichermittel 8 Beabstandungselemente 28 auf, die zwischen den einzelnen Vorhaltebereichen 32 des Latentwärmespeichermittels 8 ausgebildet sind bzw. anordenbar sind. Bevorzugt sind die Beabstandungselemente 28 auf die Wandung des Latentwärmespeichermittels 8 aufgeklebt. Es ist jedoch ebenfalls denkbar, dass die Beabstandungselemente 28 als Teil der Wandung des Latentwärmespeichermittels 8 ausgebildet sind. Weiterhin ist denkbar, dass durch mindestens ein Beabstandungsmittel 28 zumindest zwei Vorhaltebereiche kommunizierend miteinander verbunden sind.

Weiterhin ist denkbar, dass einzelne Wandungsteile des Latentwärmespeichermittels 8, insbesondere der einzelnen Vorhaltebereiche 32, durch Formhalteelemente 33 oder Formvorgabeelemente miteinander verbunden sind, wobei ein Formhalteelement 33 oder ein Formvorgabeelement bevorzugt eine Ausdehnung des durch die Wandungen lokal begrenzten Volumens begrenzt. Es wird durch die Formhalteelemente 33 somit bevorzugt verhindert, dass das flüssige Phasenwechselmaterial aus einem Vorhaltebereich heraus in einen anderen Vorhaltebereich hinein förderbar ist und somit die Menge an Phasenwechselmaterial in einem Vorhaltebereich signifikant erhöht und in einem anderen Vorhaltebereich signifikant sinkt. Es wird somit durch die Formhalteelemente 33, die z.B. lokale Verklebungen oder Verschweißungen von einen Vorhaltebereich begrenzenden Wandungsanteilen sein können, bewirkt, dass in jedem Vorhaltebereich im Wesentlichen die gewünschte Menge an Phasenwechselmaterial vorhanden ist. Weiterhin kann der Fig. 9a entnommen werden, dass durch die Beabstandungselemente 28 ein Wärmeübertragungsbereich 30 geschaffen wird, der sich innerhalb des äußeren Umfangs des Latentwärmespeichermittels 8 befindet. Der Wärmeübertagungsbereich 30 wird im Verwendungsfall zumindest teilweise durch das temperierte fließfähige Material gefüllt.

In Fig. 9b ist eine weitere denkbare Ausführungsform des Latentwärmespeichermittels 8 gezeigt. Gemäß dieser Ausführungsform kann das Latentwärmespeichermittel 8 mehrere gleichförmige und bevorzugt ebenfalls mehrere davon verschieden ausgebildete Kammern 24, 26 aufweisen, wobei bevorzugt alle Kammern 24, 26 kommunizierend miteinander verbunden sind. Weiterhin lässt sich der Fig. 9b entnehmen, dass der Wärmeübertragungsbereich 30 zwischen den einzelnen Vorhaltebereichen 32 durch die Form der einzelnen Kammern 24, 26 gebildet wird. Wobei das Volumen des Innenraums der Latentwärmespeichermittel 8 bevorzugt aus den durch die Vorhaltebereiche 32 begrenzten Volumina besteht.

Die in den Figuren 9a und 9b gezeigten Latentwärmespeichermittel 8 sind idealisiert dargestellt im Zentrum der Wärmeeinrichtung 1 angeordnet. In einem Verwendungszustand liegen sie jedoch bevorzugt an einem Abschnitt der den Aufnahmeraum 2 begrenzenden Wandung 6 an. Weiterhin ist bzgl. der in den Figuren 9a und 9b gezeigten Latentwärmespeichermittel 8 denkbar, dass diese miteinander kombiniert werden bzw. zwei oder mehrere unterschiedliche Latentwärmespeichermittel 8 in einer Wärmeeinrichtung verwendet werden bzw. ein mindestens ein Latentwärmespeichermittel 8 geschaffen wird, das Eigenschaften mehrere hier (nicht nur bzgl. der Figuren 9a und 9b) beschriebener Latentwärmespeichermittel 8 aufweist.

Die Figuren 9a und 9b zeigen somit jeweils ein Latentwärmespeichermittel 8, das in einer Wärmeeinrichtung 1, insbesondere einer Wärmflasche, verwendbar ist. Das Latentwärmespeichermittel 8 umfasst dabei bevorzugt mindestens einen durch eine flexible Wandung begrenzten Innenraum zum Aufnahmen eines Phasenwechselmaterials, wobei das Phasenwechselmaterial bei einer endothermen Zustandsänderung Energie aufnimmt und bei einer exothermen Zustandsänderung Energie in Form von Wärme abgibt, wobei die flexible Wandung derart ausgebildet ist, das ein erster Anteil des Innenraums sich in einer ersten Ebene erstreckt und ein zweiter Anteil des Innenraums sich zumindest abschnittsweise und bevorzugt vollständig in einer zweiten Ebene erstreckt, wobei die erste Ebene und die zweite Ebene parallel zueinander sind, und wobei zwischen dem ersten Anteil des Innenraums und dem zweiten Anteil des Innenraums ein Wärmeübertragungsbereich ausbildbar ist oder ausgebildet ist, über den von einem temperierten fließfähigen Material Wärme auf das Phasenwechselmaterial übertragbar ist. Die Wandung des Latentwärmespeichermittels 8 besteht bevorzugt teilweise und bevorzugt vollständig aus einem wasserundurchlässigen Werkstoff, insbesondere einem Polymer und/oder einer Membran.

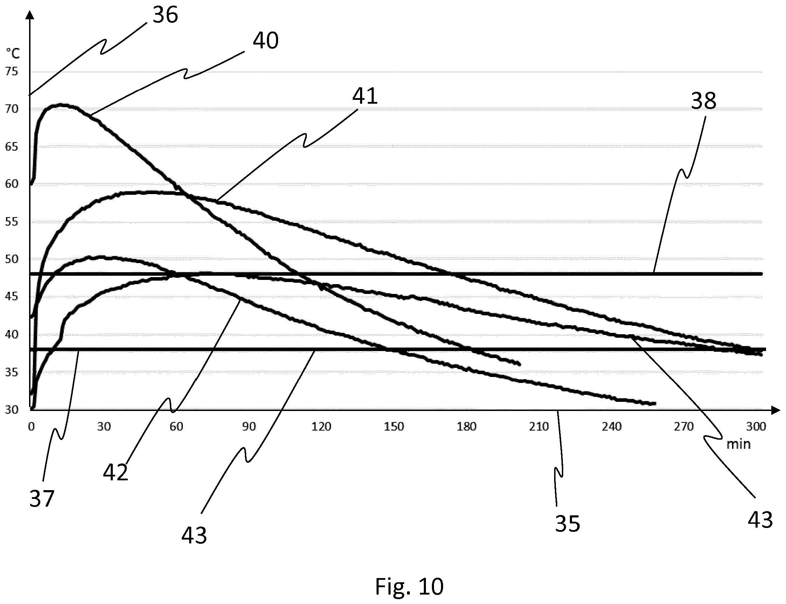

Fig. 10 zeigt ein Diagramm gemäß dem auf der X-Achse 35 die Zeit in Minuten angegeben ist und auf der Y-Achse 36 die Temperatur in °C angegeben ist. Ferner kennzeichnet die Linie 37 eine untere Grenze eines optimalen Wärmebereichs und die Linie 38 eine obere Grenze eines optimalen Wärmebereichs. Die untere Grenze des optimalen Wärmebereichs liegt in dieser Darstellung bei 38°C und die obere Grenze des optimalen Wärmebereichs liegt in dieser Darstellung bei 48°C. Die Bezugszeichen 40, 41, 42, 43 kennzeichnen Abkühlkurven verschiedener Wärmflaschenkonfigurationen, wobei die Randbedingen identisch bzw. zumindest vergleichbar sind, d.h. die gleiche Wärmflaschenform, das gleiche Wärmflaschenmaterial, die gleiche Raumtemperatur, das gleiche Messverfahren und eine gleiche Aufheizung des in die Wärmflasche eingefüllten Wassers (eingebracht in die Wärmflasche mit 80°C) vorlag. Das Diagramm zeigt, dass die klassische Wärmflasche ohne Ummantelung (Abkühlkurve 40) eine Oberflächentemperatur aufweist, die gegenüber einer erfindungsgemäßen Wärmflasche ohne Ummantelung (Abkühlkurve 42) ca. doppelt solange oberhalb der oberen Grenze 38 des optimalen Wärmebereichs liegt. Bei einer klassischen Wärmflasche mit einer Ummantelung (Abkühlkurve 41), wie einer Neopren- und/oder Fleecehülle, liegt die Oberflächentemperatur der Wärmflasche sogar noch deutlich länger über der oberen Grenze 38 des optimalen Temperaturbereichs. Es ist somit ersichtlich, dass bei gemäß den Kurven 40 und 41 abkühlenden Wärmflaschen eine große Verbrennungsgefahr besteht. Weiterhin kann dem Diagramm entnommen werden, dass die erfindungsgemäße Wärmeinrichtung, insbesondere Wärmflasche, mit einer Ummantelung, insbesondere einer Neopren- und/oder Fleeceummantelung, am längsten im optimalen Wärmebereich liegt, insbesondere um mindestens den Faktor 2 länger im optimalen Wärmebereich liegt als die anderen getesteten Varianten.

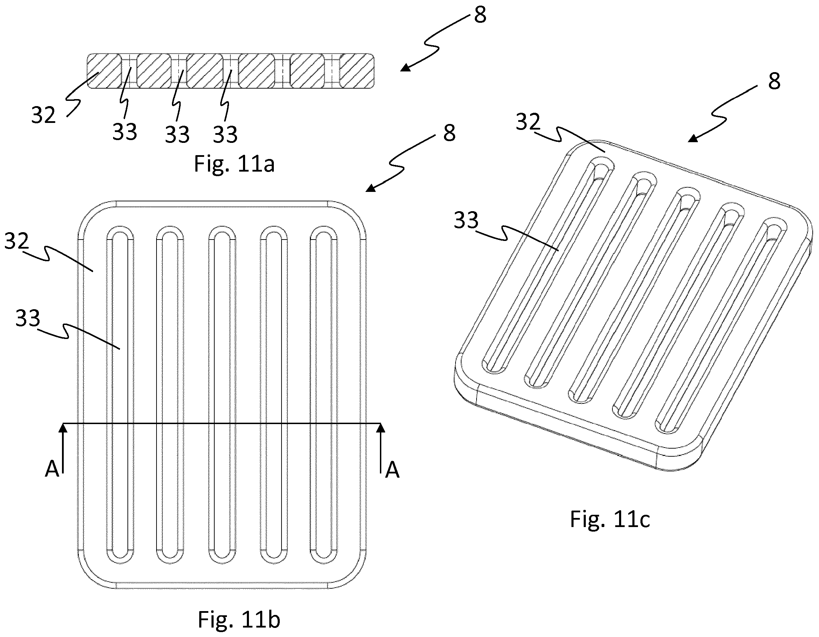

Fig. 11a zeigt einen Querschnitt eines Latentwärmespeichermittels 8. Der Querschnitt korrespondiert mit dem in Fig. 11b durch den Buchstaben A gekennzeichneten Schnitt. Das Bezugszeichen 32 kennzeichnet hierbei den Vorhaltebereich, in dem das Phasenwechselmaterial aufgenommen bzw. vorgehalten wird. Das Bezugszeichen 33 kennzeichnet dabei ein Formhalteelement, das in der gezeigten Darstellung eine strebenartige Ausgestaltung des Latentwärmespeichermittels 8 bewirkt. Durch das Formhalteelement 33 wird eine Verformung des Latentwärmespeichermittels 8 derart verhindert, dass sich das Latentwärmespeichermittel 8 nicht infolge von Verlagerungen des Phasenwechselmaterials lokal ausbeult. Weiterhin bewirken die Formhalteelemente 33, dass die Oberfläche des den Vorhaltebereich 32 begrenzenden Latentwärmespeichermittels größer ist also ohne diese Formhalteelemente 33.

Fig. 11b zeigt eine Draufsicht auf das Latentwärmespeichermittel 8 und Fig. 11c zeigt eine perspektivische Ansicht des Latentwärmespeichermittels 8.

Gemäß einzelner der in den Figuren 1 bis 11c dargestellten Ausführungsformen kann das Latentwärmespeichermittel 8 zusätzlich oder alternativ bevorzugt derart ausgebildet sein, dass bei einer definierten Menge, insbesondere einer vollen Füllung des Aufnahmeraums 2, an definiert temperiertem, insbesondere mit 95°C eingefüllten, fließfähigem Material, insbesondere Wasser, die endotherme Zustandsänderung des Phasenwechselmaterials vollständig bewirkbar ist.

Gemäß einzelner der in den Figuren 1 bis 11c dargestellten Ausführungsformen kann das Latentwärmespeichermittel 8 zusätzlich oder alternativ bevorzugt ein Betätigungsmittel 10, insbesondere einen bevorzugt mindestens ein Metall aufweisenden Knackfrosch, zum Auslösen der exothermen Zustandsänderung aufweisen.

Gemäß einzelner der in den Figuren 1 bis 11c dargestellten Ausführungsformen kann das Latentwärmespeichermittel 8 zusätzlich oder alternativ bevorzugt ein Betätigungsmittel 10, insbesondere einen bevorzugt mindestens zwei Metalle aufweisende Metalleinrichtung, insbesondere eine Bi-Metalleinrichtung, wie z.B. ein Bi-Metallstreifen, zum selbsttätigen Auslösen der exothermen Zustandsänderung aufweisen. Bevorzugt ist das Betätigungsmittel 10 derart ausgebildet, dass es in Abhängigkeit von der Temperatur eine Verformung erfährt. Dies ist vorteilhaft, da das Betätigungsmittel 10 so beim Abkühlen eines in die flüssige Form überführten Phasenwechselmaterials auf eine Temperatur unterhalb der Erstarrungstemperatur des Phasenwechselmaterials durch eine Formänderung einen Phasenwechsel des Phasenwechselmaterials, insbesondere durch Keimbildung oder durch Keimfreisetzung, selbsttätig auslöst.

Gemäß einzelner der in den Figuren 1 bis 11c dargestellten Ausführungsformen kann das Latentwärmespeichermittel 8 zusätzlich oder alternativ derart ausgebildet sein, dass bei einer definierten Menge, insbesondere einer vollen Füllung des Aufnahmeraums 2, an definiert temperiertem, insbesondere mit 95°C eingefülltem, fließfähigem Material, insbesondere Wasser, die endotherme Zustandsänderung des Phasenwechselmaterials nur teilweise bewirkbar ist.

Die Erfindung bezieht sich somit auf eine Wärmeeinrichtung 1, insbesondere eine Wärmflasche oder ein Wärmekissen, zum zumindest mittelbaren in Kontakt bringen mit einem Lebewesen. Die Wärmeeinrichtung umfasst mindestens einen Aufnahmeraum 2 zum Vorhalten eines fließfähigen Materials 4, wobei der Aufnahmeraum 2 zumindest abschnittsweise durch eine flexible Wandung 6 ausgebildet ist, wobei die flexible Wandung 6 zumindest mittelbar mit dem Lebewesen in Kontakt bringbar ist. Erfindungsgemäß ist ein Latentwärmespeichermittel 8 vorgesehen, wobei das Latentwärmespeichermittel derart angeordnet ist, dass zumindest zeitweise durch das Latentwärmespeichermittel 8 eine Temperierung des fließfähigen Materials 4 bewirkbar ist, wobei das Latentwärmespeichermittel 8 ein Phasenwechselmaterial, insbesondere Natriumacetat, aufweist, wobei das Phasenwechselmaterial bei einer endothermen Zustandsänderung infolge einer Erwärmung Energie aufnimmt und bei einer exothermen Zustandsänderung Energie in Form von Wärme abgibt. Das fließfähige Material wird dabei bevorzugt mit einer Temperatur von mehr als 60°C, 70°C, 80°C, 90°C und bis zu 95°C, insbesondere mit einer Temperatur zwischen 60°C und 100°C oder zwischen 70°C und 100°C oder zwischen 80°C und 100°C oder zwischen 90°C und 100°C in die Wärmeeinrichtung eingebracht oder darin auf diese Temperatur temperiert. Wobei die Füllung der Wärmeeinrichtung mit der fließfähigen Substanz bevorzugt zwischen 40% und 100% liegt, insbesondere zwischen 50% und 100% oder 60% und 100% oder 70% und 100% oder 80% und 100% oder 90% und 100%. Wobei das Latentwärmespeichermittel derart gestaltet ist bzw. soviel Phasenwechselmaterial vorgesehen ist, dass das Phasenwechselmaterial bei der zuvor genannten Temperatur der fließfähigen Substanz und der zuvor genannten Füllmenge an Wasser und bevorzugt bei einer Umgebungstemperatur von 20°C oder von 25 °C oder von 30°C oder von 40°C nicht vollständig oder nur teilweise aus einem ersten stabilen Zustand, in dem das Phasenwechselmaterial bevorzugt festkörperartig ist oder fest ist, in einen zweiten Zustand, in dem das Phasenwechselmaterial flüssig ist, überführbar ist.

Das Phasenwechselmaterial ist bevorzugt ein anorganisches Material, insbesondere ein Material auf Salzbasis. Besonders bevorzugt ist das Phasenwechselmaterial ein Material das aus einem ersten stabilen Aggregatszustand (fest) in einen zweiten meta-stabilen Aggregatszustand (flüssig) überführbar ist.

Bevorzugt ist das Latentwärmespeichermittel, insbesondere wenn kein Betätigungsmittelt darin vorgesehen ist, derart ausgebildet, dass die darin vorgehaltenen Phasenwechselmaterialanteile funktional zusammenwirken, insbesondere ein in einem Materialanteil des Phasenwechselmaterials vorhandener Restkeim den exothermen Phasenwechsel des gesamten Phasenwechselmaterials bewirkt. Besonders bevorzugt ist das Latentwärmespeichermittel somit derart ausgebildet ist, dass die darin vorgehaltenen Phasenwechselmaterialanteile funktional zusammenwirken, wobei ein in einem Materialanteil des Phasenwechselmaterials vorhandener Keim den exothermen Phasenwechsel des gesamten Phasenwechselmaterials auslöst, wenn die Temperatur des fließfähigen Materials unter die Erstarrungstemperatur oder Schmelztemperatur des Phasenwechselmaterials fällt. Das Phasenwechselmaterial ist bevorzugt in der Ausführungsform ohne Betätigungsmittel in einer solchen Menge oder Masse vorgesehen, dass bei einem zumindest zu 2/3 mit auf eine Temperatur oberhalb des Schmelzpunktes des Phasenwechselmaterials temperierten fließfähigen Materials gefüllten Aufnahmeraum die endotherme Zustandsänderung des Phasenwechselmaterials bei einer Umgebungstemperatur von 20°C nur teilweise bewirkbar ist. In anderen Worten: Das Phasenwechselmaterial ist in einer solchen Menge oder Masse vorgesehen, dass die endotherme Zustandsänderung des Phasenwechselmaterials nur teilweise bewirkbar ist, wenn der Aufnahmeraum bei einer Umgebungstemperatur von 20°C zumindest zu 2/3 mit dem temperierten fließfähigen Material gefüllt ist und wenn das fließfähige Material auf eine Temperatur oberhalb des Schmelzpunktes des Phasenwechselmaterials erhitzt ist.

**Bezugszeichenliste**

| | | | |
|---|---|---|---|
| 1 | Wärmeeinrichtung | 36 | Y-Achse zum Darstellen der Temperatur |
| 2 | Aufnahmeraum | 37 | Untere Temperaturgrenze des optimalen |
| 4 | Fließfähiges Material | | Temperaturbereichs |
| 6 | Flexible Wandung | 38 | Obere Temperaturgrenze des optimalen Temperaturbereichs |
| 8 | Latentwärmespeichermittel | 40 | Abkühlkurve einer klassischen mit Wasser befüllten Wärmflasche |
| 9 | Innenraum des Latentwärmespeichermittels | | |
| | | 41 | Abkühlkurve der klassischen Wärmflasche plus Neoprenbezug |
| 10 | Betätigungsmittel | | |
| 12 | Zuführ- und Entleeröffnung | 42 | Abkühlkurve der erfindungsgemäßen |
| 14 | Verschluss | | Wärmflasche |
| 15 | Trichter | 43 | Abkühlkurve der erfindungsgemäßen Wärmflasche mit Neopren bezug |
| 16 | Markierung | A | Schnitt |
| 18 | Abgrenzeinrichtung | T | Temperatur |
| 20 | gewöhnliche Abkühkurve | X | erste Richtung / Länge |
| 22 | modifizierte Abkühlkurve | Y | zweite Richtung / Tiefe |
| 24 | Hauptkammer | Z | dritte Richtung / Höhe |
| 26 | Nebenkammer | a | Starttemperatur |
| 28 | Beabstandungselement | b | Konstanttemperatur |
| 30 | Wärmeübertragungsbereich | c | Endtemperatur |
| 32 | Vorhaltebereich | g | Schwerkraft |
| 33 | Formhalteelement | | |
| 35 | X-Achse zum Darstellen der Zeit | t | Zeit |

## Patentansprüche

1. Wärmflasche (1) zum zumindest mittelbaren in Kontakt bringen mit einem Lebewesen,
mindestens umfassend
einen Aufnahmeraum (2) zum Vorhalten eines fließfähigen Materials (4), wobei an dem Aufnahmeraum (2) eine verschließbare Zuführ- und Entleeröffnung (12) zum Zuführen des fließfähigen Materials (4) in den Aufnahmeraum (2) und zum Ausleiten des, insbesondere erkalteten, fließfähigen Materials (4) aus dem Aufnahmeraum (2) heraus ausgebildet ist,
wobei der Aufnahmeraum (2) zumindest abschnittsweise durch eine flexible Wandung (6) ausgebildet ist,
wobei die flexible Wandung (6) zumindest mittelbar mit dem Lebewesen in Kontakt bringbar ist,
und ein Latentwärmespeichermittel (8),
wobei das Latentwärmespeichermittel derart angeordnet ist, dass zumindest zeitweise durch das Latentwärmespeichermittel (8) eine Temperierung des fließfähigen Materials (4) bewirkbar ist,
wobei das Latentwärmespeichermittel (8) innerhalb des Aufnahmeraums (2) angeordnet oder als Teil der Wandung (6) ausgebildet ist
wobei das Latentwärmespeichermittel (8) ein Phasenwechselmaterial aufweist, wobei das Phasenwechselmaterial bei einer endothermen Zustandsänderung infolge einer Erwärmung Energie aufnimmt und bei einer exothermen Zustandsänderung Energie in Form von Wärme abgibt,
wobei das Phasenwechselmaterial in einer solchen Menge oder Masse vorgesehen ist, dass bei einem zumindest zu 2/3 mit auf eine Temperatur oberhalb des Schmelzpunktes des Phasenwechselmaterials temperierten fließfähigen Materials gefüllten Aufnahmeraum die endotherme Zustandsänderung des Phasenwechselmaterials bei einer Umgebungstemperatur von 20°C nur teilweise bewirkbar ist, wobei das fließfähige Material eine Flüssigkeit ist,
**dadurch gekennzeichnet, dass**
das Phasenwechselmaterial zumindest Salzbestandteile aufweist, wobei die Schmelztemperatur des Phasenwechselmaterials zwischen 36°C und 70°C liegt,
wobei das Phasenwechselmaterial Natriumacetat-Trihydrat aufweist,
wobei einzelne Wandungsteile des Latentwärmespeichermittels (8) durch Formhalteelemente (33) oder Formvorgabeelemente miteinander verbunden sind, wobei ein Formhalteelement (33) oder ein Formvorgabeelement eine Ausdehnung des durch die Wandungen lokal begrenzten Volumens begrenzt.

2. Wärmflasche (1) nach einem der vorangegangenen Ansprüche,
**dadurch gekennzeichnet, dass**
an dem Aufnahmeraum (2) eine verschließbare Zuführ- und Entleeröffnung (12) zum Zuführen des, insbesondere erhitzten, fließfähigen Materials (4) zu dem Aufnahmeraum (2) und zum Ausleiten des, insbesondere erkalteten, fließfähigen Materials (4) aus dem Aufnahmeraum (2) heraus ausgebildet ist.

3. Wärmflasche (1) nach einem der vorangegangenen Ansprüche,
**dadurch gekennzeichnet, dass**
das Latentwärmespeichermittel (8) innerhalb des Aufnahmeraums (2) oder an der Wandung (6) des Aufnahmeraums (2) angeordnet ist oder als Teil der Wandung (6) ausgebildet ist.

4. Wärmflasche (1) nach einem der Ansprüche 1 bis 3,
**dadurch gekennzeichnet, dass**
das Verhältnis zwischen der Menge oder Masse des in dem Aufnahmeraum (2) aufnehmbaren fließfähigen Materials und der Menge oder Masse des Phasenwechselmaterials zwischen 2:1 und 7:1, bevorzugt zwischen 2,5:1 und 6:1 und besonders bevorzugt zwischen 2,5:1 und 5:1 liegt.

5. Herstellverfahren zum Herstellen einer Wärmflasche (1) nach Anspruch 1 zum zumindest mittelbaren in Kontakt bringen mit einem Lebewesen,
mindestens umfassend die Schritte:
Bereitstellen zweier Rohlingsplatten, wobei durch die Rohlingsplatten die Wandung der Wärmflasche erzeugt wird,
Anordnen mindestens eines Latentwärmespeichermittels (8) zwischen den Rohlingsplatten, wobei das Latentwärmespeichermittel (8) ein Phasenwechselmaterial aufweist,
wobei das Phasenwechselmaterial zu überwiegenden Masseanteilen aus einem Salz oder mehreren Salzen besteht,
wobei das Phasenwechselmaterial bei einer endothermen Zustandsänderung Energie aufnimmt und bei einer exothermen Zustandsänderung Energie in Form von Wärme abgibt, wobei das Phasenwechselmaterial Natriumacetat-Trihydrat aufweist,
wobei einzelne Wandungsteile des Latentwärmespeichermittels (8) durch Formhalteelemente (33) oder Formvorgabeelemente miteinander verbunden sind, wobei ein Formhalteelement (33) oder ein Formvorgabeelement eine Ausdehnung des durch die Wandungen lokal begrenzten Volumens begrenzt,
Verbinden der Rohlingsplatten miteinander.

6. Latentwärmespeichermittel zur Einbringung durch eine Öffnung in eine Wärmflasche nach Anspruch 1, wobei die Öffnung einen Öffnungsdurchmesser von kleiner oder gleich 30 mm hat, wobei die Wärmflasche mindestens 0,3 Liter fließfähiges Material aufnehmen kann und mindestens eine Wanddicke von 0,5 mm aufweist,
mindestens umfassend
eine Wandung, die eine äußere dreidimensionale Form des Latentwärmespeichermittels vorgibt, wobei durch die Wandung ein Innenraum (9) begrenzt wird, in dem sich ein Phasenwechselmaterial befindet, wobei das Phasenwechselmaterial bei einer endothermen Zustandsänderung Energie aufnimmt und bei einer exothermen Zustandsänderung Energie in Form von Wärme abgibt,
wobei das Phasenwechselmaterial eine Schmelztemperatur von mehr als 36°C aufweist und in einer Menge oder Masse vorgesehen ist, die bei einer zumindest 2/3 Füllung der Wärmflasche mit mindestens 70°C heißem fließfähigen Material bei einer Umgebungstemperatur von 20°C die endotherme Zustandsänderung nur teilweise erfährt, wobei das fließfähige Material eine Flüssigkeit ist,
**dadurch gekennzeichnet, dass**
wobei das Phasenwechselmaterial zumindest Salzbestandteile aufweist,
wobei das Phasenwechselmaterial Natriumacetat-Trihydrat aufweist,
wobei einzelne Wandungsteile des Latentwärmespeichermittels (8) durch Formhalteelemente (33) oder Formvorgabeelemente miteinander verbunden sind, wobei ein Formhalteelement (33) oder ein Formvorgabeelement eine Ausdehnung des durch die Wandungen lokal begrenzten Volumens begrenzt.

7. Latentwärmespeichermittel nach Anspruch 6,
wobei die Wandung flexibel ausgebildet ist und ein erster Anteil des Innenraums sich in einer ersten Ebene erstreckt und ein zweiter Anteil des Innenraums sich zumindest abschnittsweise und bevorzugt vollständig in einer zweiten Ebene erstreckt, wobei die erste Ebene und die zweite Ebene parallel zueinander sind, und wobei zwischen dem ersten Anteil des Innenraums und dem zweiten Anteil des Innenraums ein Wärmeübertragungsbereich ausbildbar ist oder ausgebildet ist, über den von einem temperierten fließfähigen Material Wärme auf das Phasenwechselmaterial übertragbar ist.

8. Set aus einer Wärmflasche gemäß einem der Ansprüche 1 bis 4 und einer austauschbaren Ummantelung, wobei die Ummantelung zumindest die flexible Wandung der Wärmflasche abschnittsweise umschließt.

9. Set nach Anspruch 8,
**dadurch gekennzeichnet, dass**
ein Verschluss zum Öffnen und Verschließen der Wärmflasche mit umfasst ist.

## Claims

1. Hot-water bottle (1) for at least indirectly bringing into contact with a living being,
comprising at least
a receiving space (2) for holding a flowable material (4), wherein a closable supply and discharge opening (12) is formed on the receiving space (2) for supplying the flowable material (4) into the receiving space (2) and for discharging the, in particular cooled, flowable material (4) out of the receiving space (2),
wherein the receiving space (2) is formed at least in sections by a flexible wall (6),
wherein the flexible wall (6) can be brought at least indirectly into contact with the living being and a latent heat storage means (8),
wherein the latent heat storage means is arranged in such a way that a tempering of the flowable material (4) can be effected at least temporarily by the latent heat storage means (8),
wherein the latent heat storage means (8) is arranged within the receiving space (2) or is formed as part of the wall (6)
wherein the latent heat storage means (8) comprises a phase change material, wherein the phase change material absorbs energy during an endothermic change of state due to heating and releases energy in the form of heat during an exothermic change of state,
wherein the phase change material is provided in such a quantity or mass that, with a receiving space filled at least 2/3 with flowable material tempered to a temperature above the melting point of the phase change material, the endothermic change of state of the phase change material is only partially effectable at an ambient temperature of 20°C, wherein the flowable material is a liquid,
**characterized in that**
the phase change material comprises at least salt constituents, wherein the melting temperature of the phase change material is between 36°C and 70°C,
wherein the phase change material comprises sodium acetate trihydrate,
wherein individual wall parts of the latent heat storage means (8) are connected to one another by shape retaining elements (33) or shape presetting elements, wherein a shape retaining element (33) or a shape presetting element limits an expansion of the volume locally bounded by the walls.

2. Hot-water bottle (1) according to any one of the preceding claims,
**characterized in that**
a closable feed and discharge opening (12) for feeding the, in particular heated, flowable material (4) to the receiving space (2) and for discharging the, in particular cooled, flowable material (4) out of the receiving space (2) is formed on the receiving space (2).

3. Hot-water bottle (1) according to one of the preceding claims,
**characterized in that**
the latent heat storage means (8) is arranged within the receiving space (2) or on the wall (6) of the receiving space (2) or is formed as part of the wall (6).

4. Hot-water bottle (1) according to any one of claims 1 to 3,
**characterized in that**
the ratio between the amount or mass of the flowable material receivable in the receiving space (2) and the amount or mass of the phase change material is between 2:1 and 7:1, preferably between 2.5:1 and 6:1 and particularly preferably between 2.5:1 and 5:1.

5. Manufacturing method for manufacturing a hot-water bottle (1) according to claim 1 for at least indirectly bringing into contact with a living being,
at least comprising the steps of:
Providing two blank plates, the blank plates creating the wall of the hot-water bottle,
arranging at least one latent heat storage means (8) between said blank plates, said latent heat storage means (8) comprising a phase change material
wherein the phase change material consists of one or more salts in predominant mass proportions,
wherein the phase change material absorbs energy during an endothermic change of state and releases energy in the form of heat during an exothermic change of state,
wherein the phase change material comprises sodium acetate trihydrate,
wherein individual wall parts of the latent heat storage means (8) are connected to each other by shape retaining elements (33) or shape presetting elements, wherein a shape retaining element (33) or a shape presetting element limits an expansion of the volume locally bounded by the walls,
joining the blank plates to one another.

6. Latent heat storage means for insertion through an opening in a hot-water bottle according to claim 1, wherein the opening has an opening diameter of less than or equal to 30 mm, wherein the hot-water bottle can hold at least 0.3 liters of flowable material and has at least a wall thickness of 0.5 mm,
comprising at least
a wall defining an outer three-dimensional shape of the latent heat storage means, the wall defining an inner space (9) in which a phase change material is located, the phase change material absorbing energy during an endothermic change of state and releasing energy in the form of heat during an exothermic change of state,
wherein the phase change material has a melting temperature of more than 36°C and is provided in a quantity or mass which only partially undergoes the endothermic change of state when the hot-water bottle is at least filled 2/3 full with flowable material having a temperature of at least 70°C at an ambient temperature of 20°C, wherein the flowable material is a liquid,
**characterized in that**
wherein the phase change material comprises at least salt constituents,
wherein the phase change material comprises sodium acetate trihydrate,
wherein individual wall parts of the latent heat storage means (8) are connected to each other by shape retaining elements (33) or shape presetting elements, wherein a shape retaining element (33) or a shape presetting element limits an expansion of the volume locally bounded by the walls.

7. Latent heat storage means according to claim 6,
wherein the wall is formed flexibly and a first portion of the interior extends in a first plane and a second portion of the interior extends at least in sections and preferably completely in a second plane, wherein the first plane and the second plane are parallel to each other, and wherein between the first portion of the interior and the second portion of the interior a heat transfer region can be formed or is formed, via which heat can be transferred from a tempered flowable material to the phase change material.

8. Set comprising a hot-water bottle according to any one of claims 1 to 4 and a replaceable sheath, the sheath enclosing at least a portion of the flexible wall of the hot-water bottle.

9. Set according to claim 8,
**characterized in that**
a closure for opening and closing the hot-water bottle is included.

## Revendications

1. Bouteille chauffante (1) pour le contact au moins indirect avec un être vivant,
comprenant au moins
un espace de logement (2) pour contenir un matériau fluide (4), dans lequel, au niveau de l'espace de logement (2), est réalisée une ouverture d'introduction et de vidange fermable (12) pour l'introduction du matériau fluide (4) dans l'espace de logement (2) et pour l'évacuation du matériau fluide (4), plus particulièrement refroidi, hors de l'espace de logement (4),
dans lequel l'espace de logement (2) est constitué, au moins à certains endroits, d'une paroi flexible (6),
dans lequel la paroi flexible (6) peut être mise en contact au moins indirectement avec l'être vivant,
et un moyen d'accumulation de chaleur latente (8),
dans lequel le moyen d'accumulation de chaleur latente est disposé de sorte qu'une régulation de la température (4) par le moyen d'accumulation de chaleur latente (8) est possible au moins temporairement,
dans lequel le moyen d'accumulation de chaleur latente (8) est disposé à l'intérieur de l'espace de logement (2) ou est réalisé en tant que partie de la paroi (6),
dans lequel le moyen d'accumulation de chaleur latente (8) comprend un matériau à changement de phase, dans lequel le matériau à changement de phase absorbe de l'énergie lors d'un changement d'état endotherme à la suite d'un échauffement et dégage de l'énergie sous forme de chaleur lors d'un changement d'état exotherme,
dans lequel le moyen d'accumulation de chaleur latente est prévu dans une quantité ou masse telle que, dans le cas d'un espace de logement rempli, à au moins 2/3, d'un matériau fluide régulé en température supérieure au point de fusion du matériau à changement de phase, le changement d'état endotherme du matériau de changement de phase est possible uniquement partiellement à une température ambiante de 20 °C, dans lequel le matériau fluide est un liquide,
**caractérisé en ce que**
le matériau de changement de phase comprend au moins des composants de type sels, dans lequel la température de fusion du matériau à changement de phase est entre 36 °C et 70 °C,
dans lequel le matériau à changement de phase comprend de l'acétate de sodium trihydraté,
dans lequel différentes parties de la paroi du moyen d'accumulation de chaleur latente (8) sont reliées entre eux par des éléments de maintien de forme (33) ou des éléments de prédétermination de forme, dans lequel un élément de maintien de forme (33) ou un élément de prédétermination de forme limite une dilatation du volume limité localement par les parois.

2. Bouteille chauffante (1) selon l'une des revendications précédentes,
**caractérisé en ce que**
au niveau de l'espace de logement (2), est réalisée une ouverture d'introduction et de vidange fermable (12) pour l'introduction du matériau fluide (4), plus particulièrement chauffé, dans l'espace de logement (2) et pour l'évacuation du matériau fluide (4), plus particulièrement refroidi, hors de l'espace de logement (2).

3. Bouteille chauffante (1) selon l'une des revendications précédentes,
**caractérisé en ce que**
le moyen d'accumulation de chaleur latente (8) est disposé à l'intérieur de l'espace de logement (2) ou au niveau de la paroi (6) de l'espace de logement (2) ou est réalisé en tant que partie de la paroi (6).

4. Bouteille chauffante (1) selon l'une des revendications 1 à 3,
**caractérisé en ce que**
le rapport entre la quantité ou la masse du matériau fluide pouvant être logé dans l'espace de logement et la quantité ou la masse du matériau à changement de phase est entre 2:1 et 7:1, de préférence entre 2,5:1 et 6:1 et plus particulièrement de préférence entre 2,5:1 et 5:1.

5. Procédé de fabrication pour la fabrication d'une bouteille chauffante (1) selon la revendication 1 pour la mise en contact ou moins indirecte avec un être vivant,
comprenant au moins les étapes suivantes :
la mise en disposition de deux plaques d'ébauche, dans lequel les plaques d'ébauche permettent de produire la paroi de la bouteille chauffante,
la disposition d'au moins un moyen d'accumulation de chaleur latente (8) entre les plaques d'ébauche, dans lequel le moyen d'accumulation de chaleur latente (8) comprend un matériau à changement de phase,
dans lequel le matériau à changement de phase est constitué, en majeure partie, d'un sel ou de plusieurs sels,
dans lequel le matériau à changement de phase absorbe de l'énergie lors d'un changement d'état endotherme et dégage de l'énergie sous forme de chaleur lors d'un changement d'état exotherme,
dans lequel le matériau à changement de phase comprend de l'acétate de sodium trihydraté,
dans lequel différentes parties de paroi du moyen d'accumulation de chaleur latente (8) sont reliés entre eux par des éléments de maintien de forme (33) ou des éléments de prédétermination de forme, dans lequel un élément de maintien de forme (33) ou un élément de prédétermination de forme limite une dilatation du volume limité localement par les parois,
raccordement des plaques d'ébauche entre elles.

6. Moyen d'accumulation de chaleur latente destiné à être introduit à travers une ouverture dans une bouteille chauffante selon la revendication 1,
dans lequel l'ouverture présente un diamètre d'ouverture inférieure ou égale à 30 mm, dans lequel la bouteille chauffante peut contenir au moins 0,3 litre de matériau fluide et présente une épaisseur de paroi d'au moins 0,5 mm,
comprenant au moins
une paroi qui prédétermine une forme tridimensionnelle externe du moyen d'accumulation de chaleur latente, dans lequel la paroi délimite un espace interne (9) dans lequel se trouve un matériau à changement de phase, dans lequel le matériau à changement de phase absorbe de l'énergie lors d'un changement d'état endotherme et dégage de l'énergie sous forme de chaleur lors d'un changement d'état exotherme,
dans lequel le matériau à changement de phase présente une température de fusion supérieure à 36 °C et est prévu dans une quantité ou masse qui, lors d'un remplissage au 2/3 de la bouteille chauffante avec un matériau fluide chauffé à au moins 70 °C, à une température ambiante de 20 °C, est soumis au changement d'état endotherme uniquement partiellement, dans lequel le matériau fluide est un liquide,
**caractérisé en ce que**
dans lequel le matériau à changement de phase comprend au moins des composants de types sels,
dans lequel le matériau à changement de phase comprend de l'acétate de sodium trihydraté,
dans lequel différentes parties de paroi du moyen d'accumulation de chaleur latentes sont reliées entre elles par des éléments de maintien de forme (33) ou des éléments de prédétermination de forme, dans lequel un élément de maintien de forme (33) ou un élément de prédétermination de forme limite une dilatation du volume limité localement par les parois.

7. Moyen d'accumulation de chaleur latente selon la revendication 6,
dans lequel la paroi est réalisée de manière flexible et une première partie de l'espace interne s'étend dans un premier plan et une deuxième partie de l'espace interne s'étend au moins à certains endroits et de préférence entièrement dans un deuxième plan, dans lequel le premier plan et le deuxième plan sont parallèles entre eux, et dans lequel, entre la première partie de l'espace interne et la deuxième partie de l'espace interne, une zone de transmission de chaleur peut être réalisée ou est réalisée, par l'intermédiaire de laquelle le matériau fluide régulé en température permet de transmettre de la chaleur au matériau à changement de phase.

8. Kit constitué d'une bouteille chauffante selon l'une des revendications 1 à 4 et une enveloppe interchangeable, dans lequel l'enveloppe entoure la paroi flexible de la bouteille chauffante au moins à certains endroits.

9. Kit selon la revendication 8,
**caractérisé en ce que**
une fermeture pour l'ouverture et la fermeture de la bouteille chauffante est inclus dans le kit.
